(19) Europäisches Patentamt
European Patent Office
Office européen des brevets

(11) Publication number: **0 297 534 B1**

(12) **EUROPEAN PATENT SPECIFICATION**

(45) Date of publication of patent specification: **28.12.94**

(51) Int. Cl.[5]: **C07D 471/04**, C07H 17/02, A61K 31/435, A61K 31/70, //(C07D471/04,221:00,209:00)

(21) Application number: **88110367.5**

(22) Date of filing: **29.06.88**

The file contains technical information submitted after the application was filed and not included in this specification

(54) **Castanospermine esters and glycosides.**

(30) Priority: **02.07.87 US 69351**

(43) Date of publication of application:
**04.01.89 Bulletin 89/01**

(45) Publication of the grant of the patent:
**28.12.94 Bulletin 94/52**

(84) Designated Contracting States:
**AT BE CH DE ES FR GB GR IT LI LU NL SE**

(56) References cited:

**CHEMICAL ABSTRACTS, vol. 100, no. 23, June 4, 1984, Columbus, Ohio, USA RONALD C. BERNOTAS et al. "Total syntheses of (+)--castanospermine and (+)-deoxynojirimycin" page 607, column 2, Abstract-no. 192 129b**

**CHEMICAL ABSTRACTS, vol. 105, no. 3, July 21, 1986, Columbus, Ohio, USA HIROYUKI SETOI et al. "Total synthesis of (+)-castanospermine from D-mannose" page 662, column 1, Abstract-no. 24 481v**

(73) Proprietor: **MERRELL DOW PHARMACEUTICALS INC.**
**2110 East Galbraith Road**
**Cincinnati**
**Ohio 45215-6300 (US)**

(72) Inventor: **Liu, Paul S.**
**7370 Drake Road**
**Cincinnati**
**Ohio 45243 (US)**
Inventor: **Daniel, John K.**
**7303 Back Bay Court**
**Indianapolis**
**Indiana 46214 (US)**
Inventor: **Rhinehart, Barry L.**
**10921 Thornview Drive**
**Cincinnati**
**Ohio 45241 (US)**

(74) Representative: **Sgarbi, Renato et al**
**GRUPPO LEPETIT S.p.A.**
**Patent and Trademark Department**
**Via Roberto Lepetit, 34**
**I-21040 Gerenzano (Varese) (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 297 534 B1

CHEMICAL ABSTRACTS, vol. 107, no. 23, December 7, 1987, Columbus, Ohio, USA BRUCE C. CAMPBELL et al. "Differential inhibition by castanospermine of various insect disaccharidases" page 364, column 2, Abstract-no. 215 128m

CHEMICAL ABSTRACTS, vol. 102, no. 15, April 15, 1985, Columbus, Ohio, USA RICK SAUL et al. "Castano- spermine inhibits alpha- -glucosidase activities and alters glycogen distribution in animals" page 177, column 1, Abstract-no. 126 770u

CHEMICAL ABSTRACTS, vol. 98, no. 17, April 25, 1983, Columbus, Ohio, USA RICK SAUL et al. "Castanospermine, a tetrahydroxy-lated alkaloid that inhibits beta-glucosidase and beta-glucocerebrosidase" page 255, column 2, Abstract-no. 139 631y

CHEMICAL ABSTRACTS, vol. 103, no. 3, July 22, 1985, Columbus, Ohio, USA GRAZYNA PALMARCZYK et al. "The effects of castanospermine on the oligosaccha- ride structures of glyco- proteins from lymphoma cell lines" page 400, column 2, Abstract-no. 20 519z

CHEMICAL ABSTRACTS, vol. 105, no. 15, October 13, 1986, Columbus, Ohio, USA JAMES P. CHAMBERS et al. "Effects of castanospermine on purified alpha-1,4-gluco-sidase" page 308, column 2, Abstract-no. 129 836t

CHEMICAL ABSTRACTS, vol. 95, no. 23, Dec. 7, 1981, Columbus, Ohio, USA LIZA D. HOHENSCHUTZ et al. "Castanospermine, a 1,6,7,8-tetrahydroxyoctahydroindolizine alkaloid, from seeds of Castanospermum australe" page 712, column 1, Abstract-no. 202 446f

Trugnan et al. (1986) FEBS Lett. 195, 28-32M Ariens, E.J. ed. Drug Design IX, Academic Press, NY 1980; Roche, E.B. ed; Design of Diopharmaceutical Properties through Prodrugs and Analogs; Washington DC, 1977

## Description

Castanospermine is an alkaloid which has been isolated from the seeds of Castanospermum australe and it has the following formula:

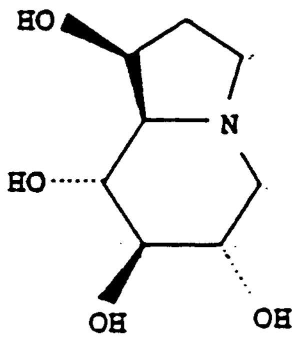

Systematically, this compound can be named in several ways as follows: [1S-(1α,6β,7α,8β,8aβ)]-octahydro-1,6,7,8-indolizinetetrol or (1S,6S,7R,8R,8aR)-1,6,7,8-tetrahydroxy-indolizidine or 1,2,4,8-tetradeoxy-1,4,8-nitrilo-*L-glycero-D-galacto*-octitol. The term "castanospermine" or the first systematic name will be used in the discussion below.

The isolation of this compound and the determination of its structure have been described by L.D. Hohenshutz, et al., Phytochemistry, 20, 811 (1981). As part of his study of castanospermine, Hohenshutz obtained castanospermine tetraacetate by the reaction of castanospermine with a very large excess of acetic anhydride but there is no suggestion of any other esters of castanospermine in the article.

The present invention is directed to certain esters and glycoside derivatives of castanospermine. More particularly, it is directed to compounds having the following general formula:

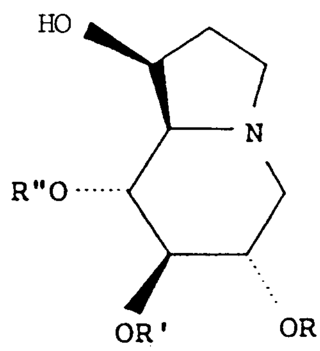

wherein R, R' and R" are independently hydrogen, $C_{1-10}$ alkanoyl, cyclohexanecarbonyl,

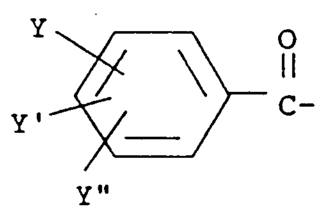 ,

naphthalenecarbonyl optionally substituted by methyl or halogen; phenyl($C_{2-6}$ alkanoyl) wherein the phenyl is optionally substituted by methyl or halogen; cinnamoyl; pyridinecarbonyl optionally substituted by methyl or halogen; thiophenecarbonyl optionally substituted by methyl; furancarbonyl optionally substituted by methyl; or a glycosyl, an *O*-methylglycosyl or Ac-acylated glycosyl radical containing from 1 to 3 hexose or

pentose units with attachment at the 1-position of the glycosyl radical;
Y is hydrogen, $c_{1-4}$ alkyl, $C_{1-4}$ alkoxy, halogen, trifluoromethyl, $C_{1-4}$ alkylsulfonyl, $C_{1-4}$ alkylmercapto, cyano or dimethylamino; Y' is hydrogen, $C_{1-4}$ alkyl, $C_{1-4}$ alkoxy,halogen or it is combined with Y to give 3,4-methylenedioxy; Y'' is hydrogen, $C_{1-4}$ alkyl, $C_{1-4}$ alkoxy or halogen; Ac is benzoyl or $C_{2-6}$ alkanoyl; with R, R' and R'' being selected in such a way that at least one of them, but not more than two of them, is hydrogen; or the pharmaceutically acceptable salts of the aforesaid compounds.

The $C_{1-10}$ alkanoyl groups referred to above can be straight- or branched-chain and can be exemplified by formyl, acetyl, propionyl, butyryl, isobutyryl, hexanoyl, octanoyl and decanoyl. The halogens referred to above can be exemplified by fluorine, chlorine, bromine or iodine. The $C_{2-6}$ alkanoyl groups referred to above (Ac) can be exemplified by acetyl, propionyl, butyryl, isobutyryl, and hexanoyl. The $C_{1-4}$ alkyl groups referred to above, whether alone or as part of an alkoxy, an alkylsulfonyl or an alkylmercapto group, can be straight- or branched-chain alkyl groups containing up to 4 carbon atoms. Examples of various such groups are methyl, ethyl, propyl, butyl, methoxy, ethoxy, butoxy, methylsulfonyl, ethylsulfonyl, methylmercapto and ethylmercapto. The phenyl ($C_{2-6}$ alkanoyl) groups referred to above can be exemplified by benzeneacetyl and benzenepropionyl. The various naphthalenecarbonyl, pyridinecarbonyl, thiophenecarbonyl and furancarbonyl groups referred to above include the various position isomers and these can be exemplified by naphthalene-1-carbonyl, naphthalene-2-carbonyl, nicotinoyl, isonicotinoyl, thiophene-2-carbonyl, thiophene-3-carbonyl, furan-2-carbonyl and furan-3-carbonyl. The naphthalene, pyridine, thiophene and furan groups can be optionally further substituted as indicated above.

Specific examples of the various glycosyl radicals are glucosyl, galactosyl, fucosyl, ribosyl, 2-deoxyglucosyl, 3-*O*-methylglucosyl, cellobiosyl, maltobiosyl, maltotriosyl, cellotriosyl, arabinosyl and xylosyl. Particularly preferred are the compounds wherein R is 1-glucosyl, 1-*L*-fucosyl or 1-cellobiosyl.

Acid addition salts with pharmaceutically acceptable acids referred to above are equivalent to the amines for the purposes of this invention. Illustrative of such salts are the salts with inorganic acids such as, for example, hydrochloric, hydrobromic, sulfuric, phosphoric and like acids; with organic carboxylic acids such as, for example, acetic, propionic, glycolic, lactic, pyruvic, malonic, succinic, fumaric, malic, tartaric, citric, ascorbic, maleic, hydroxymaleic and dihydroxymaleic, benzoic, phenylacetic, 4-aminobenzoic, 4-hydroxybenzoic, anthranilic, cinnamic, salicylic, 4-aminosalicylic, 2-phenoxybenzoic, 2-acetoxybenzoic, mandelic and like acids; and with organic sulfonic acids such as methanesulfonic acid and p-toluenesulfonic acid. Such salts can be obtained by standard procedures from an amine of this invention and the appropriate acid.

A preferred group of compounds are those wherein R, R' and R'' are 1 or 2 alkanoyl or benzoyl groups with the benzoyl substituted by Y, Y' and Y'' as described above.

The esters of the present invention are prepared by the reaction of castanospermine with an appropriate acid halide or anhydride in an inert solvent. The halide can be a chloride or bromide and the anhydride includes mixed anhydrides. The relative amount of the acid halide or anhydride used, the relative amount of solvent, the temperature and the reaction time are all controlled so as to minimize the number of hydroxy groups that will be acylated. Thus, only a limited excess of the acid derivative is used, which means up to about a three-fold excess of the acylating agent. Use of a solvent in relatively large amounts, serves to dilute the reactants and hold down the amount of higher acylated products that form. The solvent used is preferably one that will dissolve the reactants used without reacting with them. It is further preferable to carry out the reaction in the presence of a tertiary amine which will react with and remove any acid formed during the course of the reaction. The tertiary amine can be added to the mixture or it can itself be used in excess and serve as the solvent. Pyridine is a preferred solvent in this regard. As indicated above, the time and the temperature are likewise controlled to limit the amount of acylation that takes place. Preferably, the reaction is carried out with cooling in an ice-bath for a period of about 16 hours to give the monoesters with the reaction time extended to a longer period, such as 7 days, if diesters are desired. The reaction can actually be carried out at higher temperatures and, in fact, heating can be used as long as the various factors involved are properly controlled. The fact of the matter is, when the reaction is carried out as described, the final reaction mixture will still contain a considerable amount of unreacted castanospermine. This unreacted material can be recovered from the reaction mixture and recycled in subsequent reactions and thus increase the overall amount of castanospermine converted to ester. This recycling is particularly important when the reaction is carried out under conditions which would favor the isolation of monoesters.

The procedures as described above will generally give 6- or 7-monoesters or 6,7- or 6,8-diesters. Other isomers can be obtained by appropriate use of blocking groups. Thus, for example, castanospermine can be reacted with 2-(dibromomethyl)benzoyl chloride to give the 6,7-diester. This diester is then reacted with an appropriate acid halide or anhydride to give the corresponding 8-ester. The two protecting groups are then readily removed by conversion of the two dibromomethyl groups to formyl (using silver perchlorate

and 2,4,6-collidine in aqueous acetone) followed by hydrolysis of the formylbenzoic acid ester obtained using morpholine and hydroxide ion. The indicated procedure can be used in a similar way to give diester isomers.

To obtain the glycoside derivatives of the present invention, castanospermine or an esterified castanospermine is reacted with an appropriate glycosyl halide or an appropriate glycosyl acetimidate, wherein the glycosyl hydroxy groups are protected as the Ac-esters or with benzyl groups, in an inert solvent followed, if necessary, by catalytic hydrogenation to remove any benzyl groups. The inert solvent used can be a halogenated hydrocarbon such as methylene chloride. The glycosyl halide can be a chloride or a bromide; a preferred acetimidate is trichloroacetimidate.

When an esterified castanospermine is used in the glycoside process, this serves to block any esterified hydroxide groups so that reaction must take place at another, free hydroxy group. Once the coupling of the castanospermine ester with the glycosyl derivative has taken place, any ester groups, either on the glycoside or on the castanospermine nucleus, can be removed by hydrolysis with base, for example, a strong base such as sodium methoxide in methanol. Such a hydrolysis is usually carried out before the catalytic debenzylation process referred to above.

The present compounds are useful in the treatment of diabetes. More specifically, they can be used to prevent the development of excessive hyperglycemia which may be observed in certain diabetic conditions when a glucose precursor is ingested. Thus, when carbohydrate is ingested either as glucose or in a form such as maltose, sucrose or starch in food or drink, the serum glucose level rises to elevated concentrations. In healthy subjects, this hyperglycemic state quickly returns to normal, the glucose in the blood being rapidly metabolized and stored and/or utilized by the organism. In diabetes mellitus, however, the glucose tolerance of the patient is lowered and the abnormally high serum glucose levels which develop remain elevated for prolonged periods of time. A similar response to that seen in man can also be observed in other animals, including livestock, poultry, pet animals and laboratory animals. Such a condition can be described as postprandial hyperglycemia. One method for treating such a condition would be by administration of some agents which would prevent the conversion of complex sugars to glucose and thus prevent the development of the excessive glucose levels. In the present invention, it has been found that, where the high levels of glucose are a result of the hydrolysis of complex sugars, administration of the present castanospermine derivatives inhibits the initial formation of glucose in the blood and thus makes it possible to avoid the problems which would be associated with prolonged high levels of serum glucose.

The mechanism whereby this result is achieved is the following although the utility described above should not be limited by the precise details of this mechanism. Enzymes which catalyze the hydrolysis of complex carbohydrates convert non-absorbable carbohydrate into absorbable sugars. The rapid action of these enzymes leads to acute and undesirable elevations in serum glucose in diabetes. The compounds of the present invention are potent inhibitors of these enzymes and, when co-administered with a carbohydrate meal, they prevent harmful hyperglycemic excursions of this type. It is desirable, however, that the inhibition of these hydrolytic enzymes be limited to those present in the intestines and that is true for the present compounds. Otherwise, inhibition of systemic glycohydrolases can lead to difficulty in the utilization of intracellular carbohydrates as an energy-source and thus cause metabolic problems. The first enzyme described above is intestinal sucrase whereas the second enzyme is intracellular lysosomal α-glucosidase. The compounds of the present invention were tested for activity against these enzymes by the following procedures.

Intestinal Sucrase

Sucrase was isolated from rat intestine as a crude homogenate using the salt extraction procedure of Kolinska [Biochem. Biophys. Acta, 284, 235 (1984)]. Incubation mixtures contained 100 µl of enzyme preparation plus test compound and were incubated for 1 hour before the addition of 6.6 µmole sucrose in 100 µl 0.1 M sodium maleate, pH 5.9. The mixtures were incubated an additional 30 minutes and then heat inactivated at 80-100 °C for 3 minutes. Denatured protein was removed by centrifugation. Glucose liberated was determined by glucose dehydrogenase reagents (Seragen Diagnostics).

Lysosomal α-Glucosidase

Lysosomal α-glucosidase was isolated and partially purified from rat liver by the method of Dissous [Anal. Biochem., 116, 35 (1981)] through the ammonium sulfate fractionation steps. Enzyme was incubated with test compound for 1 hour at 37 °C prior to the addition of p-nitrophenyl-α-*D*-glucoside in a final volume of 0.6 ml of 0.1 M sodium acetate, 25 mM potassium chloride, pH 4.2. Mixtures were incubated for an

additional 30 minutes at 37°C and then heat inactivated at 90°C. Denatured protein was removed by centrifugation. One ml of 0.1 M sodium carbonate was added to the supernatant fraction and liberated nitrophenol determined by its absorption at 410 nm.

The results observed when the compounds of the present invention were tested as described above can be summarized as follows:

TABLE I

| Castanospermine Derivative | $IC_{50}$ | |
|---|---|---|
| | Intestinal Sucrase (M) | Lysosomal α-Glucosidase (M) |
| 6-Benzoate | $8 \times 10^{-8}$ | $8 \times 10^{-4}$ |
| 6-(4-Fluorobenzoate) | $2 \times 10^{-7}$ | $4 \times 10^{-6}$ |
| 6-(4-Methylbenzoate) | $3 \times 10^{-7}$ | $1 \times 10^{-3}$ |
| 6-(4-Methoxybenzoate) | $2 \times 10^{-6}$ | $1 \times 10^{-3}$ |
| 7-Benzoate | $2 \times 10^{-6}$ | $2 \times 10^{-4}$ |
| 7-(4-Fluorobenzoate) | $4 \times 10^{-7}$ | $6 \times 10^{-6}$ |
| 7-(2,4-Dichlorobenzoate) | $4 \times 10^{-7}$ | $1 \times 10^{-4}$ |
| 6,7-Dibenzoate | $2 \times 10^{-6}$ | $> 10^{-3}$ |
| 6,7-bis(4-Fluorobenzoate) | $4 \times 10^{-6}$ | $> 10^{-3}$ |
| 6,8-Dibutyrate | $2 \times 10^{-7}$ | $> 10^{-3}$ |
| 8-α-*D*-Glucopyranoside | $5 \times 10^{-8}$ | $3 \times 10^{-4}$ |

From the above results, it can be seen that the present compounds have a lower $IC_{50}$ against intestinal sucrase than against lysosomal α-glucosidase.

The compounds of the present invention were further tested in a sucrose load test to determine their ability to inhibit serum glucose elevation. The procedure can be summarized as follows.

ICR Swiss mice, fasted for 18-20 hours, were orally dosed with test compound plus sucrose at 2.0 g/kg. At 30 minutes post sucrose, the animals were sacrificed and their serum glucose concentrations were determined. The amount of test compound needed to significantly inhibit the serum glucose elevation was determined by comparison to the serum glucose concentration of animals dosed only with sucrose. To test duration of action, mice were orally dosed twice. The initial dose was test compound or vehicle. After 1, 2, or 3 hours, the mice were dosed with sucrose at 2.0 g/kg. After an additional 30 minutes post sucrose, the animals were sacrificed and their serum glucose concentrations were determined. Test compound activity was indicated by a significant difference of serum glucose concentration from the corresponding control. The activity observed can be summarized as follows:

TABLE II

| Castanospermine Derivative | Effective Dose (mg/kg) | Duration Of Action |
|---|---|---|
| 6-Benzoate | 1 | 1 hour |
| 6-(4-Fluorobenzoate) | 5 | -- |
| 7-Benzoate | 5 | -- |
| 7-(4-Fluorobenzoate) | 5 | 2 hours |
| 7-(2,4-Dichlorobenzoate) | 20 | -- |
| 6,7-Dibenzoate | 20 | -- |
| 8-α-*D*-Glucopyranoside | 1 | ≧3 hours |

In practicing the method of this invention, an amount of one of the compounds effective to inhibit postprandial hyperglycemia is administered to a mammal in need thereof by a suitable route. For the purposes of this invention, oral administration is preferred.

The effective amount of the compound, that is, the amount sufficient to inhibit postprandial hyperglycemia, depends on various factors such as the size, type and age of the animal to be treated, the particular compound or pharmaceutically acceptable salt employed, the frequency of administration, the severity of the condition and the time of administration. Generally speaking, the compounds would be administered

orally at a dose of 0.1 mpk to 50 mpk, with a dose of 0.5 mpk to 5 mpk being preferred. More specifically, the present compounds would be administered to humans in single unit doses containing 35 mg to 350 mg of active ingredient with the material being administered three times a day at mealtime.

In practicing the method of this invention, the active ingredient is preferably incorporated in a composition comprising a pharmaceutical carrier and from about 5 to about 90 percent by weight of a compound of the invention or a pharmaceutically-acceptable salt thereof. The term "pharmaceutical carrier" refers to known pharmaceutical excipients useful in formulating pharmaceutically active compounds for internal administration to animals, and which are substantially non-toxic and non-sensitizing under conditions of use. The compositions can be prepared by known techniques for the preparation of tablets, capsules, elixirs, syrups, emulsions, dispersions and wettable and effervescent powders, and can contain suitable excipients known to be useful in the preparation of the particular type of composition desired. Suitable pharmaceutical carriers and formulation techniques are found in standard texts, such as *Remington's Pharmaceutical Sciences,* Mack Publishing Company, Easton, Pennsylvania.

The following examples are presented to illustrate the present invention. However, they should not be construed as limiting it in any way.

EXAMPLE 1

A slurry of 4.0 g of castanospermine in 140 ml of pyridine was stirred at room temperature for 30 minutes until essentially all of the solids had dissolved. The solution was cooled to 0°C in an ice/water bath, and a solution of 5.85 ml of benzoyl chloride in 15 ml of pyridine was added dropwise over 15 minutes under nitrogen. After the addition, the reaction mixture was stirred at 8°C overnight.

The reaction mixture was partitioned between 225 ml methylene chloride and 300 ml water. The organic layer was separated and the aqueous layer extracted with two 225-ml portions of methylene chloride. The combined organic layers were washed successively with 150 ml of 0.5 N hydrochloric acid, saturated sodium carbonate, water and saturated sodium chloride solutions, and then dried over sodium sulfate. Evaporation of solvents under reduced pressure gave 2.9 g of a tan glassy residue.

This material was slurried in chloroform and a white precipitate formed. These solids were isolated to afford 910 mg of a white powder. Thin layer chromatography (85:15, ethyl acetate:methanol) analysis showed the material to be composed of two components (Rf 0.33 and Rf 0.26). The solid mixture was slurried in 45 ml of 4:1 ethyl acetate:methanol and filtered. The residue was dried *in vacuo* to provide 350 mg of (1S-(1α,6β,7α,8β,8aβ)]-octahydro-1,6,7,8-indolizinetetrol 6-benzoate as a white powdery solid melting at about 233-236°C, with decomposition. This corresponded to the less polar component of the mixture. NMR (DMSO-$d_6$) δ 1.5-2.2 (m, 5H), 2.9-3.6 (m, 4H), 4.1 (m, 1H, $C_1$-H), 4.3 (d, 1H, -OH) 4.7 (d, 1H, -OH), 4.8 (sextet, 1H, $C_6$-H), 5.1 (d, 1H, -OH), 7.6-8.1 (m, 5H, aryl). MS (CI-$CH_4$) 294 ($MH^+$), 276 ($MH^+$-$H_2O$), 172 ($MH^+$-$PhCO_2H$).

The filtrate from above was condensed and fractionated by preparative thin layer chromatography (silica gel, 80:20, ethyl acetate:methanol) to provide 120 mg of the more polar component, [1S-(1α,6β,7α,8β,8aβ)]-octahydro-1,6,7,8-indolizinetetrol 7-benzoate as a white powdery solid melting at about 200-202°C. NMR (DMSO-$d_6$ + $D_2O$) 1.5-2.2 (m, 5H), 2.9-3.1 (m, 2H), 3.6-3.8 (m, 2H), 4.1 (m, 1H, $C_1$-H), 4.8 (t, 1H, $C_7$-H), 7.4-8.1 (m, 5H, aryl). MS (CI-$CH_4$) 294 ($MH^+$), 276 ($MH^+$-$H_2O$), 172 ($MH^+$-$PhCO_2H$). This compound has the following structural formula:

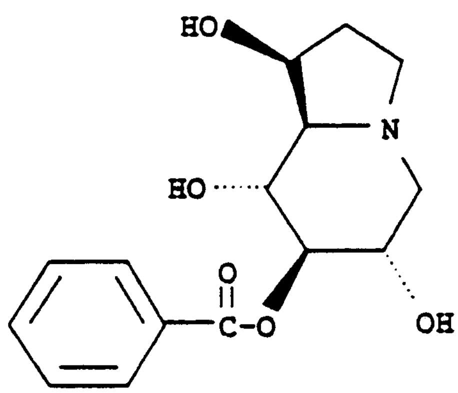

EXAMPLE 2

Castanospermine (1.89 g) was added to a stirred solution of 10 ml of pyridine and cooled to 0 °C in an ice bath. Benzoyl chloride, 3.0 g, was added dropwise to the mixture and the resulting suspension was kept at 0-4 °C for 7 days. Water, 10 ml, was added and the mixture was evaporated to dryness *in vacuo*. The resulting residue was redissolved in 1:1 water:ethyl acetate (100 ml) and the phases were separated. The aqueous layer was extracted again with 100 ml of ethyl acetate. The organic extracts were combined and concentrated to a syrup which was shown to be a mixture of two major components by thin layer chromatography (1:1 ethyl acetate:hexane, silica gel, Rf = 0.42 and Rf = 0.11). The mixture was separated by preparative high pressure liquid chromatography (silica gel, 1:1 ethyl acetate:hexane) to provide 1.9 g (48%) of the more polar [1S-(1α,6β,7α,8β,8aβ)]-octahydro-1,6,7,8-indolizinetetrol 6,7-dibenzoate as a dry foam melting at about 79-81 °C. NMR ($DMSO-d_6/D_2O$) δ 1.5-2.3 (m, 5H), 3.0-3.4 (m, 2H), 3.9 (t, 1H), 4.2 (m, 1H, $C_1$-$\underline{H}$), 5.15 (m, 1H, $C_6$-$\underline{H}$), 5.3 (t, 1H, $C_7$-$\underline{H}$), 7.4-8.0 (m, 10H, aryl). MS (FAB-Xe) 398 ($MH^+$), 380 ($MH^+$-$H_2O$), 276 ($MH^+$-$PhCO_2H$). The less polar component (Rf = 0.42) was isolated as a dry foam melting at about 75-78 °C which was [1S-(1α,6β,7α,8β,8aβ)]-octahydro-1,6,7,8-indolizinetetrol 6,7,8-tribenzoate.

EXAMPLE 3

When the procedure of Example 1 was repeated using castanospermine and the appropriate acid chloride, the following compounds were obtained:

[1S-(1α,6β,7α,8β,8aβ)]-octahydro-1,6,7,8-indolizinetetrol 6-(4-fluorobenzoate) melting at about 216-218 °C.

[1S-(1α,6β,7α,8β,8aβ)]-octahydro-1,6,7,8-indolizinetetrol 7-(4-fluorobenzoate) melting at about 190-193 °C.

[1S-(1α,6β,7α,8β,8aβ)]-octahydro-1,6,7,8-indolizinetetrol 7-(2,4-dichlorobenzoate) melting at about 179-181 °C.

[1S-(1α,6β,7α,8β,8aβ)]-octahydro-1,6,7,8-indolizinetetrol 6-(4-bromobenzoate) melting at about 234-235 °C.

[1S-(1α,6β,7α,8β,8aβ)]-octahydro-1,6,7,8-indolizinetetrol 7-(4-bromobenzoate) melting at about 199-202 °C.

(1S-(1α,6β,7α,8β,8aβ)]-octahydro-1,6,7,8-indolizinetetrol 6-(4-methoxybenzoate) melting at about 221-224 °C.

EXAMPLE 4

When the procedure of Example 2 was repeated using castanospermine and 4-fluorobenzoyl chloride, the product obtained was [1S-(1α,6β,7α,8β,8aβ)]-octahydro-1,6,7,8-indolizinetetrol 6,7-bis(4-fluorobenzoate) melting at about 82-84 °C.

EXAMPLE 5

To a suspension of 3 g of castanospermine in 30 ml of pyridine at 0 °C was added dropwise a solution of 3 g of 4-methylbenzoyl chloride. After the addition, the mixture was allowed to warm to room temperature and then heated at 55 °C for 24 hours. The reaction mixture was diluted with 10 ml of water and evaporated to dryness *in vacuo*. The resulting residue was stirred in 150 ml of a 1:2 mixture of water:methylene chloride. The insoluble material was separated by filtration to provide an amorphous off-white solid which was dissolved in 60 ml of hot methanol, treated with 0.5 g of activated charcoal and filtered. The colorless filtrate was cooled to give colorless crystals of [1S-(1α,6β,7α,8β,8aβ)]-octahydro-1,6,7,8-indolizinetetrol 6-(4-methylbenzoate) melting at about 255-258 °C with decomposition (580 mg, 12% yield).

The two-phase water/methylene chloride mixture obtained above was evaporated to dryness and the residue was dissolved in 50 ml of a 1:2 mixture of methanol:ethyl acetate. The solution was fractionated by preparative high pressure liquid chromatography (silica gel, 9:1 ethyl acetate:methanol) and fractions containing the more polar component (i.e., more polar than the 6-ester obtained in the preceding paragraph) were collected and evaporated *in vacuo* to provide a colorless solid which was [1S-(1α,6β,7α,8β,8aβ)]-octahydro-1,6,7,8-indolizinetetrol 7-(4-methylbenzoate) melting at about 220-223 °C with decomposition (210 mg, 4% yield).

EXAMPLE 6

When the procedure of Example 5 is repeated using castanospermine and the appropriate acid chloride, the following esters are obtained:

6-(2-Methylbenzoate) melting at about 213-215 °C.
6-(3-Methylbenzoate) melting at about 212 °C with decomposition.
7-(3-Methylbenzoate).
6-(3-Trifluoromethylbenzoate).
6-(4-Methylsulfonylbenzoate).
6-(4-Methylmercaptobenzoate).
6-(3-Cyanobenzoate).
6-(4-Dimethylaminobenzoate).
6-(3,4-Methylenedioxybenzoate).
6-(3,4,5-Trichlorobenzoate).
7-(3,4,5-Trichlorobenzoate).
6-(2,4-Dimethylbenzoate).
6-(2-Naphthalenecarboxylate).
7-(2-Naphthalenecarboxylate).
6-(4-Methyl-2-naphthalenecarboxylate).
6-(Benzeneacetate).
7-(Benzeneacetate).
6-(4-Chlorobenzeneacetate).
6-(Benzenepropionate).
6-(Cinnamate).
7-(Cinnamate).
6-(Cyclohexanecarboxylate).
6-Nicotinoate.
6-Isonicotinoate.
6-(2-Thiophenecarboxylate) melting at about 214-215 °C.
6-(2-Furancarboxylate) melting at about 209-212 °C.

EXAMPLE 7

Castanospermine (350 mg) was added to 5 ml of pyridine and stirred under nitrogen at room temperature. Butyric anhydride (0.97 g) was added dropwise and the mixture was kept at room temperature for 24 hours. The reaction mixture was evaporated to dryness *in vacuo* to leave a syrupy residue. The residue was dissolved in ether and a colorless solid precipitated when pentane was added. Recrystallization of the solid from a mixture of ether and petroleum ether gave colorless needles of [1S-(1α,6β,7α,8β,8aβ)]-octahydro-1,6,7,8-indolizinetetrol 6,8-dibutanoate melting at about 110-111 °C (22 mg, 4% yield). NMR ($CDCl_3$) δ 3.7 (t, 1H, $C_7$-H), 4.1 (m, 1H, $C_1$-H), 4.85 (t, 1H, $C_8$-H), 5.0 (m, 1H, $C_6$-H). MS (CI-$CH_4$) 330 ($MH^+$), 312 ($MH^+$-$H_2O$).

EXAMPLE 8

When the procedure of Example 7 is repeated using acetic anhydride, propionic anhydride or caproic anhydride in place of the butyric anhydride, the corresponding 6,8-diesters are obtained.

EXAMPLE 9

To a stirred solution of 1.7 g of [1S-(1α,6β,7α,8β,8aβ)]-octahydro-1,6,7,8-indolizinetetrol 6,7-dibenzoate in 60 ml of methylene chloride under nitrogen and cooled at -30 °C was added 3.8 g of 2,3,4,6-tetra-*O*-(phenylmethyl)-α-*D*-glucopyranosyl) trichloroacetimidate [prepared according to the procedure of R.R. Schmidt and J. Michel, Angew. Chem. Int. Ed. Engl., 19, 731-32 (1980)]. This was followed by dropwise addition of 1.4 g of boron trifluoride etherate. The mixture was kept at -10 °C for 72 hours and then washed successively with 60 ml of aqueous sodium bicarbonate solution and 60 ml of brine. The organic extract was concentrated *in vacuo* to give a thick syrup. Thin layer chromatography analysis (silica gel, 4:6 ethyl acetate:hexane) indicated that a less polar material of Rf 0.31 had formed. The mixture was fractionated on preparative high pressure liquid chromatography (silica gel, 4:6 ethyl acetate:hexane as eluent) to provide

810 mg (21%) of the protected glucoside product and 670 mg (39%) of recovered starting diester. Recrystallization of the product from ether/methanol gave colorless crystals of 6,7-di-*O*-benzoyl-8-*O*(2,3,4,6-tetra-*O*-(phenylmethyl)-α-*D*-glucopyranosyl)-[1S-(1α,6β,7α,8β,8aβ)]-octahydro-1,6,7,8-indolizinetetrol melting at about 145-147 °C. $^{13}C$ NMR (DMSO-$d_6$) δ 165.4 and 165.0 (2 x Ph<u>C</u>=O), 95.9 ($\underline{C}_{1'}$). MS (CI-$CH_4$) 920 ($MH^+$).

EXAMPLE 10

The protected glucoside product from Example 9 (460 mg) was added to a stirred solution of 20 ml of methanol under nitrogen. After addition of 5 drops of sodium methoxide solution (4.4 M in methanol) the mixture was stirred at room temperature for 18 hours. The mixture was evaporated to dryness and the residue was dissolved in 50 ml of ethyl acetate. The solution was washed twice with brine (20 ml) and evaporated *in vacuo* to leave a syrup. The syrupy residue was taken up in 8 ml of glacial acetic acid containing 100 mg of 10% Pd/C and hydrogenated on a Parr apparatus (3.5 atmospheres of hydrogen) for 72 hours. The catalyst was filtered and 100 mg of fresh 10% Pd/C was added. The mixture was further hydrogenated (1.0 atmosphere of hydrogen) at 70 °C for 6 hours. After filtering to remove the catalyst, the filtrate was evaporated to dryness and the thick residue was redissolved in 15 ml of water. The aqueous solution was washed twice with 50 ml of ether, concentrated *in vacuo* to about 3 ml and applied to an anion-exchange column (Bio-Rad AG-1-X8, 200-400 mesh, $OH^-$ form, 11 cm x 2.5 cm-id). The column was eluted with distilled water and 8 ml fractions were collected. Fractions containing the deprotected glucoside were pooled and lyophilized to provide a white powdery solid which was dissolved in methanol and triturated with acetone to give colorless crystals of 8-*O*-(α-*D*-glucopyranosyl)-[1S-(1α,6β,7α,8β,8aβ)]-octahydro-1,6,7,8-indolizinetetrol, melting at about 208-210 °C (147 mg, 83% yield). NMR ($D_2O$) δ 1.7 (m, 1H), 2.0-2.4 (m, 4H), 3.0-3.2 (m, 2H), 3.4 (t, 1H), 3.5-3.9 (m, 8H), 4.4 (m, 1H, $C_1$-<u>H</u>), 5.4 (d, 1H, $J_{1',2'}$=3.9 Hz, $C_{1'}$-<u>H</u>, anomeric proton). MS (CI-$CH_4$) 352 ($MH^+$), 334 ($MH^+$-$H_2O$). This compound has the following structural formula:

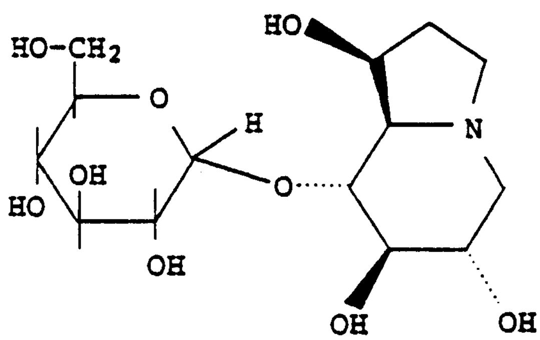

EXAMPLE 11

If the procedures of Examples 9 and 10 are repeated starting with castanospermine and the appropriate glycosyl trichloroacetimidate (obtained as indicated in Example 9), the following products are obtained:

8-*O*-(β-*D*-Glucopyranosyl)-[1S-(1α,6β,7α,8β,8aβ)]-octahydro-1,6,7,8-indolizinetetrol.

8-*O*-(β-*D*-Galactopyranosyl)-[1S-(1α,6β,7α,8β,8aβ)]-octahydro-1,6,7,8-indolizinetetrol.

8-*O*-(α-*D*-Galactopyranosyl)-[1S-(1α,6β,7α,8β,8aβ)]-octahydro-1,6,7,8-indolizinetetrol.

8-*O*-(6-Deoxy-β-*L*-galactopyranosyl)-[1S-(1α,6β,7α,8β,8aβ)]-octahydro-1,6,7,8-indolizinetetrol.

8-*O*-(6-Deoxy-α-*L*-galactopyranosyl)-[1S-(1α,6β,7α,8β,8aβ)]-octahydro-1,6,7,8-indolizinetetrol.

8-*O*-(β-*D*-Ribofuranosyl)-[1S-(1α,6β,7α,8β,8aβ)]-octahydro-1,6,7,8-indolizinetetrol.

8-*O*-(α-*D*-Ribofuranosyl)-[1S-(1α,6β,7α,8β,8aβ)]-octahydro-1,6,7,8-indolizinetetrol.

8-*O*-[α-*D*-Glucopyranosyl-(1→4)-*O*-β-*D*-glucopyranosyl]-[1S-(1α,6β,7α,8β,8aβ)]-octahydro-1,6,7,8-indolizinetetrol.

8-*O*-[α-*D*-Glucopyranosyl-(1→4)-*O*-α-*D*-glucopyranosyl]-[1S-(1α,6β,7α,8β,8aβ)]-octahydro-1,6,7,8-indolizinetetrol.

8-*O*-[β-*D*-Glucopyranosyl-(1→4)-*O*-α-*D*-glucopyranosyl]-[1S-(1α,6β,7α,8β,8aβ)]-octahydro-1,6,7,8-indolizinetetrol.

8-*O*-[β-*D*-Glucopyranosyl-(1→4)-*O*-β-*D*-glucopyranosyl]-[1S-(1α,6β,7α,8β,8aβ)]-octahydro-1,6,7,8-indolizinetetrol.

EXAMPLE 12

To a stirred suspension of 1.5 g of castonospermine in 15 ml of pyridine cooled at 0°C in an ice-bath was added dropwise 1.0 g of butyryl chloride. The mixture was stirred at room temperature for 3 days and added to a 1:1 mixture of water:methylene chloride (400 ml). After partitioning, the aqueous phase was concentrated *in vacuo* to provide an oily residue which was fractionated by radial thin layer chromatography (silica gel, 2 mm thickness plate, 2:8 methanol:chloroform) to provide 68 mg of [1S-(1α,6β,7α,8β,8aβ)]-octahydro-1,6,7,8-indolizinetetrol 6-butanoate, homogeneous by thin layer chromatography (silica gel, 2:8 methanol:chloroform, Rf = 0.5). Recrystallization of the product from 5:95 isopropanol:hexane gave a colorless solid melting at 113-114°C. NMR ($CDCl_3$) δ 3.5-3.8 (2t, 2H, $C_7$-H and $C_8$-H), 4.4 (m, 1H, $C_1$-H), 4.95 (m, 1H, $6_6$-H). MS (CI-$CH_4$) 260 ($MH^+$), 242 ($MH^+$-$H_2O$), 172 ($MH^+$-$C_3H_7CO_2H$).

Similarly, when the above procedure was repeated using acetyl chloride or propionyl chloride, the following monoesters were obtained:

[1S-(1α,6β,7α,8β,8aβ)]-octahydro-1,6,7,8-indolizinetetrol 6-acetate melting at about 188-189°C.

[1S-(1α,6β,7α,8β,8aβ)]-octahydro-1,6,7,8-indolizinetetrol 7-propionate melting at about 153-155°C.

EXAMPLE 13

To a mixture of 1.0 g of castanospermine and 30 ml of pyridine at 0°C under a blanket of nitrogen was added 12.2 g of isonicotinoyl chloride hydrochloride. The resulting yellow solution was heated at 55°C for 41 hours. A 6-mg quantity of 4-dimethylaminopyridine was added and reflux was continued for an additional 24 hours. The reaction solution was concentrated and applied to a 700-ml volume column of Kieselgel 60 and eluted with 80:20:1: chloroform:methanol:ammonium hydroxide. After collection of a 300-ml forerun, 100-ml fractions were collected. Fractions 11-21 were concentrated to afford 180 mg of material which was applied to a radial chromatography plate (2 mm: Kieselgel 60 PF-254) and eluted with 9:1: chloroform:methanol. After eluting three bands, fractions containing a fourth band were combined and concentrated to give [1S-(1α,6β,7α,8β,8aβ)]-octahydro-1,6,7,8-indolizinetetrol 7-isonicotinoate as a white solid. NMR (DMSO-$d_6$) δ 8.81 (d, J=5.9 Hz, 2H, pyridyl protons ortho to N), 7.87 (d, J=5.9 HZ, 2H, pyridyl protons meta to N), 4.85 (dd, J=9.2 Hz, 1H, C7-H).

EXAMPLE 14

Castanospermine (0.38 g) was added to a stirred solution of 5 ml of pyridine and cooled in an ice bath. Benzoyl chloride, 0.96 g, was added dropwise to the mixture and the resulting suspension was kept at 0-4°C for 18 hours. Ice water, 5 ml, was added and the mixture was diluted with 50 ml of ether. The ethereal solution was separated and washed with 1N hydrochloric acid (2 x 50 ml), saturated aqueous sodium bicarbonate solution (50 ml) and brine (50 ml). The organic phase was dried with anhydrous magnesium sulfate and evaporated to dryness *in vacuo*. The resulting residue was redissolved in 3 ml of ether and shown to be a mixture of two major components by thin layer chromatography (6:4 ether:hexane, silica gel, $R_f$ =0.35; 0.20). The mixture was separated by preparative thin layer chromatography (silica gel, 6:4 ether:hexane) to provide 0.30 g (30%) of the less polar ($R_f$ = 0.35) [1S-(1α,6β,7α,8β,8aβ)]-octahydro-1,6,7,8-indolizinetetrol 1,6,8-tribenzoate as a dry, foamy solid melting at about 85-87°C. NMR ($CDCl_3/D_2O$) δ 1.7-2.6 (m, 5H), 3.0-4.1 (m, 3H), 5.1-5.7 (m, 3H),7.1-8.2 (m, 15H, aryl). MS (CI-$NH_3$) 502 ($MH^+$), 380 ($MH^+$ - $PhCO_2H$). The more polar component ($R_f$ = 0.20) was isolated as a dry foam melting at about 75-78°C and was [1S-(1α,6β,7α,8β,8aβ)]-octahydro-1,6,7,8-indolizinetetrol 6,7,8-tribenzoate.

EXAMPLE 15

To a stirred solution of 5.2 g of [1S-(1α,6β,7α,8β,8aβ)]-octahydro-1,6,7,8-indolizinetetrol 1,6,8-tribenzoate in 150 ml of methylene chloride under nitrogen and cooled at -20°C there was added 8.5 g of 2,3,4,6-tetra-*O*-(phenylmethyl)-α-*D*-glucopyranosyl trichloroacetimidate. This was followed by dropwise addition of 3.0 ml of boron trifluoride etherate. The mixture was kept at -10°C for 96 hours. After warming to room temperature, the mixture was washed successively with 200 ml of aqueous sodium bicarbonate

solution and 200 ml of brine. The organic phase was dried with magnesium sulfate and evaported *in vacuo* to provide a thick syrup. Thin layer chromatography analysis (silica gel, 1:3 ethyl acetate:hexane) showed that a less polar material of $R_f$ = 0.44 had formed. The mixture was fractionated on preparative medium pressure liquid chromatography (silica gel, 1:3 ethyl acetate:hexane as eluent) to provide 5.7 g (54%) of 1,6,8-tri-*O*-benzoyl-7-*O*-(2,3,4,6-tetra-*O*-(phenylmethyl)-α-*D*-glucopyranosyl)-[1S-(1α,6β,7α,8β,8aβ)]-octahydro-1,6,7,8-indolizinetetrol as a thick colorless syrup. $^{13}$C NMR ($CDCl_3$) δ 166.0, 165.9, 165.5 (3 x Ph<u>C</u> = O),

$$99.4\ (C_1,\ J_{^{13}C-^{1}H} = 168\ Hz).$$

MS (CI-$CH_4$) 1024 ($MH^+$), 902 ($MH^+$ - $PhCO_2H$).

<u>EXAMPLE 16</u>

To a suspension of 5.6 g of 1,6,8-tri-*O*-benzoyl-7-*O*-(2,3,4,6-tetra-*O*-(phenylmethyl)-α-*D*-glucopyranosyl)-[1S-(1α,6β,7α,8β,8aβ)]-octahydro-1,6,7,8-indolizinetetrol in 100 ml of methanol was added 30 drops of sodium methoxide solution (4.4 M in methanol) and the mixture was stirred at room temperature for 18 hours. To the mixture was added 3 g of potassium hydroxide and 5 ml of water and the resulting solution was heated under reflux for 3 days. The mixture was cooled and evaporated to dryness *in vacuo*. The residue was dissolved in a mixture of 50 ml of ethyl acetate and 10 ml of water. After separating the phases, the aqueous layer was extracted twice with 30-ml portions of ethyl acetate. The organic extracts were combined and evaporated to provide a yellowish, gummy residue. Recrystallization of the product from ether gave 3.42 g (88%) of colorless, fluffy crystals of 7-*O*-(2,3,4,6-tetra-*O*-(phenylmethyl)-α-*D*-glucopyranosyl)-[1S-(1α,6β,7α,8β,8aβ)]-octahydro-1,6,7,8-indolizinetetrol melting at about 119-120 °C. $^{1}$H NMR ($CDCl_3$) δ 1.5-2.5 (m, 5H), 2.8-4.0 (m, 12H), 4.1-5.0 (m, 12H), 7.0-7.4 (m, 20H, aryl).

<u>EXAMPLE 17</u>

The product from Example 16 (250 mg) was dissolved in 4 ml of glacial acetic acid and 50 mg of 10% Pd/C was added. The mixture was hydrogenated (at 1.0 atmosphere of hydrogen) at 55 °C for 4 hours. After removal of the charcoal catalyst, the acetic acid solution was evaporated to dryness *in vacuo*. The resulting residue was redissolved in 10 ml of (1:1) methanol-water and stirred for 1 hour with 2.0 g of anion-exchange resin [Bio-Rad AGI-X8 (200-400 mesh, $OH^-$ form)]. The aqueous solution was evaporated to dryness to provide a white powdery solid which was dissolved in methanol and triturated with acetone to give 92 mg of colorless crystals of 7-*O*-(α-*D*-glucopyranosyl)-[1S-(1α,6β,7α,8β,8aβ)]-octahydro-1,6,7,8-indolizinetetrol, melting at about 184-187 °C (with decomposition). MS (CI-$CH_4$) 352 ($MH^+$), 334 ($MH^+$ - $H_2O$).

## Claims

### Claims for the following Contracting States : AT, BE, CH, DE, FR, GB, IT, LI, LU, NL, SE

**1.** A compound of the formula:

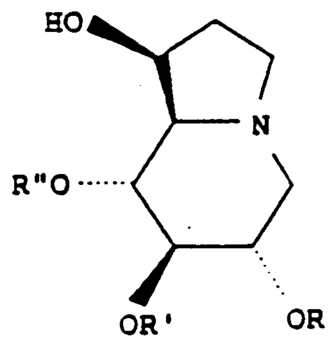

wherein R, R' and R'' are independently hydrogen, $C_{1-10}$ alkanoyl, cyclohexanecarbonyl,

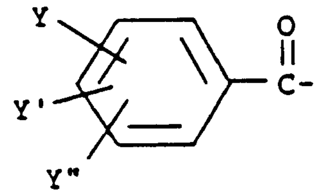 ,

naphthalenecarbonyl optionally substituted by methyl or halogen; phenyl($C_{2-6}$ alkanoyl) wherein the phenyl is optionally substituted by methyl or halogen; cinnamoyl; pyridinecarbonyl optionally substituted by methyl or halogen; thiophenecarbonyl optionally substituted by methyl; furancarbonyl optionally substituted by methyl; or a glycosyl, an *O*-methylglycosyl or Ac-acylated glycosyl radical containing from 1 to 3 hexose or pentose units with attachment at the 1-position of the glycosyl radical; Y is hydrogen, $C_{1-4}$ alkyl, $C_{1-4}$ alkoxy, halogen, trifluoromethyl, $C_{1-4}$ alkylsulfonyl, $C_{1-4}$ alkylmercapto, cyano or dimethylamino; Y' is hydrogen, $C_{1-4}$ alkyl, $C_{1-4}$ alkoxy,halogen or it is combined with Y to give 3,4-methylenedioxy; Y'' is hydrogen, $C_{1-4}$ alkyl, $C_{1-4}$ alkoxy or halogen; Ac is benzoyl or $C_{2-6}$ alkanoyl; with R, R' and R'' being selected in such a way that at least one of them, but not more than two of them, is hydrogen; or the pharmaceutically acceptable salts of the aforesaid compounds.

**2.** A compound according to Claim 1 having the formula:

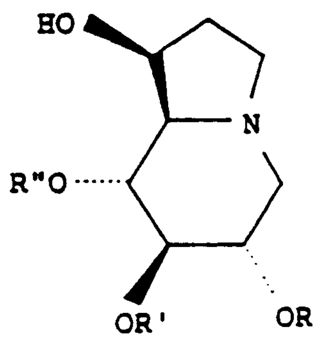

wherein R, R' and R'' are independently hydrogen, $C_{1-10}$ alkanoyl, cyclohexanecarbonyl,

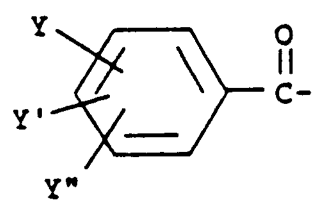 ,

naphthalenecarbonyl optionally substituted by methyl or halogen; phenyl($C_{2-6}$ alkanoyl) wherein the phenyl is optionally substituted by methyl or halogen; cinnamoyl; pyridinecarbonyl optionally substituted by methyl or halogen; thiophenecarbonyl optionally substituted by methyl; furancarbonyl optionally substituted by methyl; Y is hydrogen, $C_{1-4}$ alkyl, $C_{1-4}$ alkoxy, halogen, trifluoromethyl, $C_{1-4}$ alkylsulfonyl, $C_{1-4}$ alkylmercapto, cyano or dimethylamino; Y' is hydrogen, $C_{1-4}$ alkyl, $C_{1-4}$ alkoxy,halogen or it is combined with Y to give 3,4-methylenedioxy; Y″ is hydrogen, $C_{1-4}$ alkyl, $C_{1-4}$ alkoxy or halogen; with R, R′ and R″ being selected in such a way that at least one of them, but not more than two of them, is hydrogen; or the pharmaceutically acceptable salts of the aforesaid compounds.

**3.** A compound according to Claim 1 having the formula:

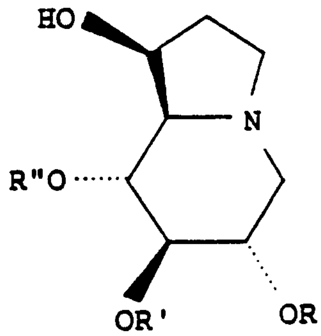

wherein R, R′ and R″ are independently hydrogen, $C_{1-10}$ alkanoyl, or

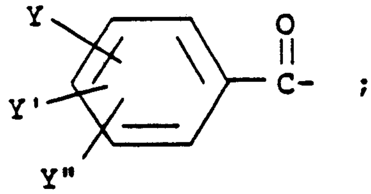

Y is hydrogen, $C_{1-4}$ alkyl, $C_{1-4}$ alkoxy, halogen, trifluoromethyl, $C_{1-4}$ alkylsulfonyl, $C_{1-4}$ alkylmercapto, cyano or dimethylamino; Y′ is hydrogen, $C_{1-4}$ alkyl, $C_{1-4}$ alkoxy, halogen or it is combined with Y to give 3,4-methylenedioxy; Y″ is hydrogen, $C_{1-4}$ alkyl, $C_{1-4}$ alkoxy or halogen; with R, R′ and R″ being selected in such a way that at least one of them, but not more than two of them is hydrogen; or the pharmaceutically acceptable salts of the aforesaid compounds.

**4.** A compound according to Claim 1 having the formula:

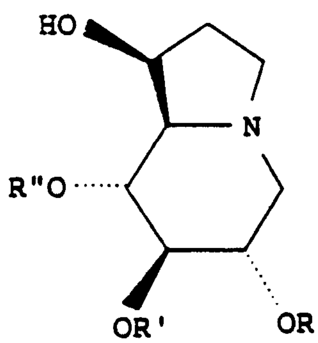

wherein R, R′ and R″ are independently hydrogen or

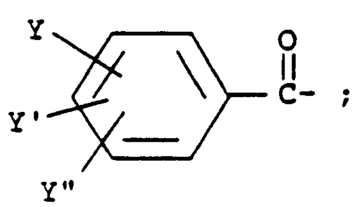

Y is hydrogen, $C_{1-4}$ alkyl, $C_{1-4}$ alkoxy, halogen, trifluoromethyl, $C_{1-4}$ alkylsulfonyl, $C_{1-4}$ alkylmercapto, cyano or dimethylamino; Y′ is hydrogen, $C_{1-4}$ alkyl, $C_{1-4}$ alkoxy, halogen or it is combined with Y to give 3,4-methylenedioxy; Y″ is hydrogen, $C_{1-4}$ alkyl, $C_{1-4}$ alkoxy or halogen; with R, R′ and R″ being selected in such a way that at least one of them, but not more than two of them, is hydrogen; or the pharmaceutically acceptable salts of the aforesaid compounds.

**5.** A compound according to Claim 1 having the formula:

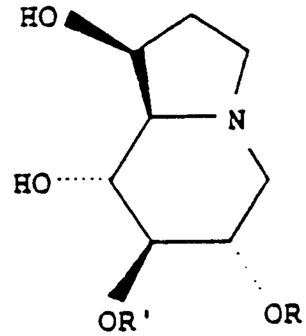

wherein R and R' are independently hydrogen or

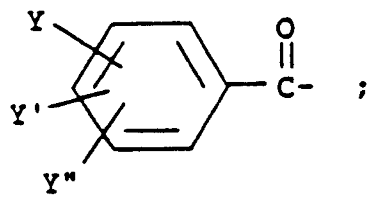

Y is hydrogen, $C_{1-4}$ alkyl, $C_{1-4}$ alkoxy, halogen, trifluoromethyl, $C_{1-4}$ alkylsulfonyl, $C_{1-4}$ alkylmercapto, cyano or dimethylamino; Y' is hydrogen, $C_{1-4}$ alkyl, $C_{1-4}$ alkoxy, halogen or it is combined with Y to give 3,4-methylenedioxy; Y'' is hydrogen, $C_{1-4}$ alkyl, $C_{1-4}$ alkoxy or halogen; with R and R' being selected in such a way that at least one of them is other than hydrogen; or the pharmaceutically acceptable salts of the aforesaid compounds.

**6.** A compound according to Claim 1 having the formula:

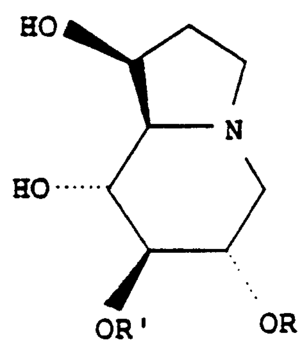

wherein R and R′ are independently hydrogen or

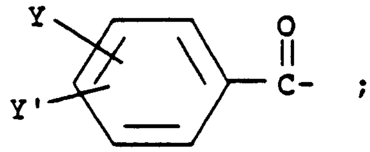

Y is hydrogen, $C_{1-4}$ alkyl, $C_{1-4}$ alkoxy, halogen, trifluoromethyl, $C_{1-4}$ alkylsulfonyl, $C_{1-4}$ alkylmercapto, cyano or dimethylamino; Y′ is hydrogen, $C_{1-4}$ alkyl, $C_{1-4}$ alkoxy, halogen or it is combined with Y to give 3,4-methylenedioxy; with R and R′ being selected in such a way that at least one of them is other than hydrogen; or the pharmaceutically acceptable salts of the aforesaid compounds.

**7.** A compound according to Claim 1 having the formula:

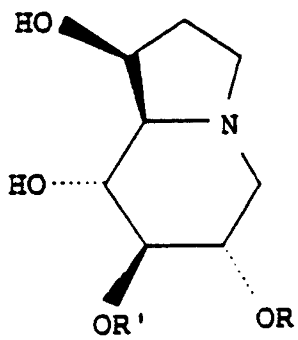

wherein R and R′ are independently hydrogen, or

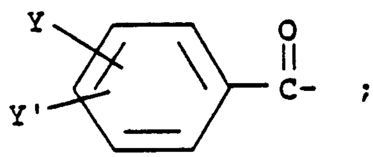

Y is hydrogen or halogen; Y′ is hydrogen or halogen; with R and R′ being selected in such a way that at least one of them is other than hydrogen; or the pharmaceutically acceptable salts of the aforesaid compounds.

**8.** A compound according to Claim 1 which is [1S-(1α,6β,7α,8β,8aβ)]-octahydro-1,6,7,8-indolizinetetrol 6-benzoate.

**9.** A compound according to Claim 1 which is [1S-(1α,6β,7α,8β,8aβ)]-octahydro-1,6,7,8-indolizinetetrol 7-benzoate.

**10.** A compound according to Claim 1 which is [1S-(1α,6β,7α,8β,8aβ)]-octahydro-1,6,7,8-indolizinetetrol 6-(4-fluorobenzoate).

**11.** A compound according to Claim 1 which is [1S-(1α,6β,7α,8β,8aβ)]-octahydro-1,6,7,8-indolizinetetrol 7-(4-fluorobenzoate).

**12.** A compound according to Claim 1 which is [1S-(1α,6β,7α,8β,8aβ)]-octahydro-1,6,7,8-indolizinetetrol 6,8-dibutanoate.

**13.** A compound according to claim 1 which is [1S-(1α,6β,7α,8β,8aβ)]-octahydro-1,6,7,8-indolizinetetrol 6-butanoate.

**14.** A compound according to Claim 1 having the formula:

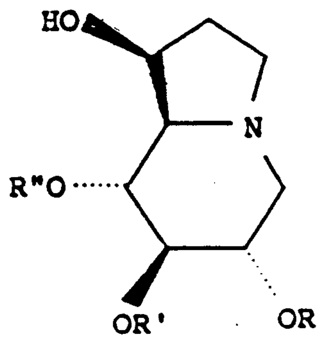

wherein R, R' and R'' are independently hydrogen, or a glycosyl, an *O*-methylglycosyl or Ac-acylated glycosyl radical containing from 1 to 3 hexose or pentose units with attachment at the 1-position of the glycosyl radical; Ac is benzoyl or $C_{2-6}$ alkanoyl; with R, R' and R'' being selected in such a way that at least one of them, but not more than two of them, is hydrogen; or the pharmaceutically acceptable salts of the aforesaid compounds.

**15.** A compound according to Claim 1 which is 8-*O*-(α-*D*-glucopyranosyl)-[1S-(1α,6β,7α,8β,8aβ)]-octahydro-1,6,7,8-indolizinetetrol.

**16.** A process for preparing a compound of claim 1 which comprises reacting castanospermine with either:
(a) the appropriate acid halide or anhydride in an inert solvent; or
(b) the appropriate Ac-acylated or *O*-benzylated glycosyl halide or acetimidate in an inert solvent followed by catalytic hydrogenation to remove the benzyl groups or optionally by treatment with base to remove the Ac-groups or other ester groups.

**17.** A compound of claim 1 for use as a medicament.

**18.** A compound of claim 2 for use as a medicament.

**19.** A compound as in any of the claims 17 and 18 for use in the treatment of diabetes in mammals.

**20.** A compound as in any of the claims 17 and 18 for use in the treatment of postprandial hyperglycemia in diabetic individuals.

**21.** A compound of claim 1 for use in the manufacture of a medicament for treating diabetes in mammals or postprandial hyperglycemia in diabetic individuals.

**22.** A compound of claim 2 for use in the manufacture of a medicament for treating diabetes in mammals or postprandial hyperglycemia in diabetic individuals.

**23.** A pharmaceutical dosage form containing a compound of claim 1.

**24.** A pharmaceutical dosage form containing a compound of claim 2.

**Claims for the following Contracting States : ES, GR**

**1.** A process for preparing a compound of the formula:

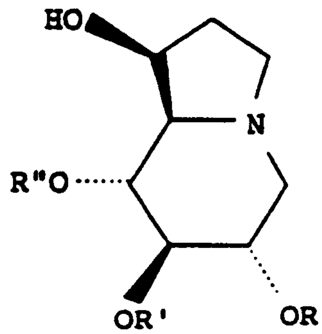

wherein R, R′ and R″ are independently hydrogen, $C_{1-10}$ alkanoyl, cyclohexanecarbonyl,

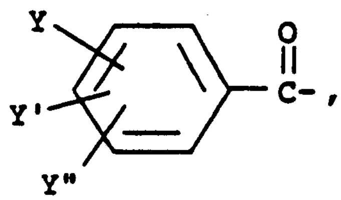

naphthalenecarbonyl optionally substituted by methyl or halogen; phenyl($C_{2-6}$ alkanoyl) wherein the phenyl is optionally substituted by methyl or halogen; cinnamoyl; pyridinecarbonyl optionally substituted by methyl or halogen; thiophenecarbonyl optionally substituted by methyl; furancarbonyl optionally substituted by methyl; or a glycosyl, an *O*-methylglycosyl or Ac-acylated glycosyl radical containing from 1 to 3 hexose or pentose units with attachment at the 1-position of the glycosyl radical; Y is hydrogen, $C_{1-4}$ alkyl, $C_{1-4}$ alkoxy, halogen, trifluoromethyl, $C_{1-4}$ alkylsulfonyl, $C_{1-4}$ alkylmercapto, cyano or dimethylamino; Y′ is hydrogen, $C_{1-4}$ alkyl, $C_{1-4}$ alkoxy, halogen or it is combined with Y to give 3,4-methylenedioxy; Y″ is hydrogen, $C_{1-4}$ alkyl, $C_{1-4}$ alkoxy or halogen; Ac is benzoyl or $C_{2-6}$ alkanoyl; with R, R′ and R″ being selected in such a way that at least one of them, but not more than two of them, is hydrogen; or the pharmaceutically acceptable salts of the aforesaid compounds, which comprises reacting castanospermine with either:

(a) the appropriate acid halide or anhydride in an inert solvent; or

(b) the appropriate Ac-acylated or *O*-benzylated glycosyl halide or acetimidate in an inert solvent followed by catalytic hydrogenation to remove the benzyl groups or optionally by treatment with base to remove the Ac-groups or other ester groups.

**2.** A process according to Claim 1 for preparing a compound of the formula

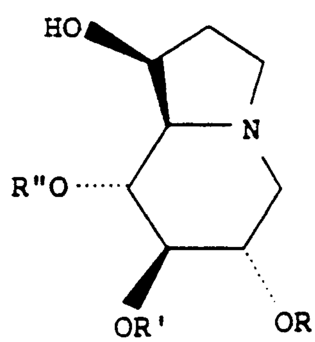

wherein R, R′ and R″ are independently hydrogen, $C_{1-10}$ alkanoyl, cyclohexanecarbonyl,

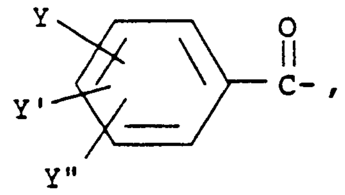

naphthalenecarbonyl optionally substituted by methyl or halogen; phenyl($C_{2-6}$ alkanoyl) wherein the phenyl is optionally substituted by methyl or halogen; cinnamoyl; pyridinecarbonyl optionally substituted by methyl or halogen; thiophenecarbonyl optionally substituted by methyl; furancarbonyl optionally substituted by methyl; Y is hydrogen, $C_{1-4}$ alkyl, $C_{1-4}$ alkoxy, halogen, trifluoromethyl, $C_{1-4}$ alkylsulfonyl, $C_{1-4}$ alkylmercapto, cyano or dimethylamino; Y′ is hydrogen, $C_{1-4}$ alkyl, $C_{1-4}$ alkoxy, halogen or it is combined with Y to give 3,4-methylenedioxy; Y″ is hydrogen, $C_{1-4}$ alkyl, $C_{1-4}$alkoxy or halogen; with R, R′ and R″ being selected in such a way that at least one of them, but not more than two of them, is hydrogen; or the pharmaceutically acceptable salts of the aforesaid compounds, which comprises reacting castanospermine with the appropriate acid halide or anhydride in an inert solvent.

3. A process according to Claim 1 for preparing a compound of the formula

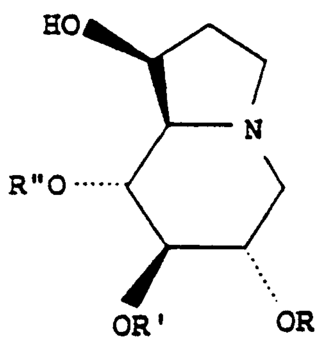

wherein R, R′ and R″ are independently hydrogen, $C_{1-10}$ alkanoyl or

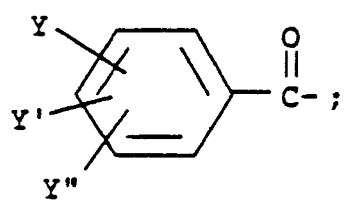

Y is hydrogen, $C_{1-4}$ alkyl, $C_{1-4}$ alkoxy, halogen, trifluoromethyl, $C_{1-4}$ alkylsulfonyl, $C_{1-4}$ alkylmercapto, cyano or dimethylamino; Y′ is hydrogen, $C_{1-4}$ alkyl, $C_{1-4}$ alkoxy, halogen or it is combined with Y to give 3,4-methylenedioxy; Y″ is hydrogen, $C_{1-4}$ alkyl, $C_{1-4}$ alkoxy or halogen; with R, R′ and R″ being selected in such a way that at least one of them, but not more than two of them, is hydrogen; or the pharmaceutically acceptable salts of the aforesaid compounds, which comprises reacting castanospermine with the appropriate acid halide or anhydride in an inert solvent.

**4.** A process according to Claim 1 for preparing a compound of the formula

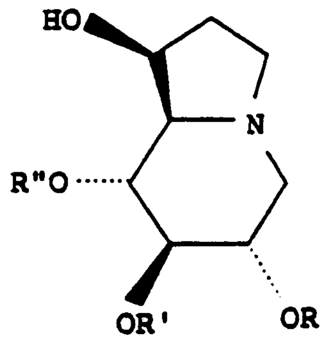

wherein R, R′ and R″ are independently hydrogen or

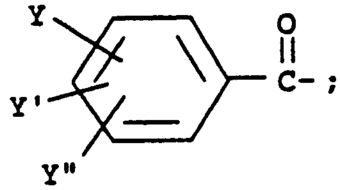

Y is hydrogen, $C_{1-4}$ alkyl, $C_{1-4}$ alkoxy, halogen, trifluoromethyl, $C_{1-4}$ alkylsulfonyl, $C_{1-4}$ alkylmercapto, cyano or dimethylamino; Y′ is hydrogen, $C_{1-4}$ alkyl, $C_{1-4}$ alkoxy, halogen or it is combined with Y to give 3,4-methylenedioxy; Y″ is hydrogen, $C_{1-4}$ alkyl, $C_{1-4}$ alkoxy or halogen; with R, R′ and R″ being selected in such a way that at least one of them, but not more than two of them, is hydrogen; or the pharmaceutically acceptable salts of the aforesaid compounds, which comprises reacting castanospermine with the appropriate acid halide or anhydride in an inert solvent.

**5.** A process according to Claim 1 for preparing a compound of the formula

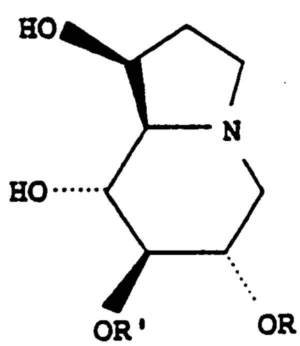

wherein R and R′ are independently hydrogen or

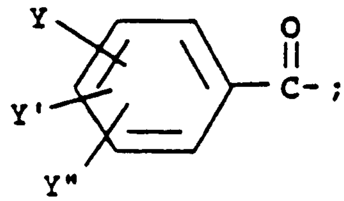

Y is hydrogen, $C_{1-4}$ alkyl, $C_{1-4}$ alkoxy halogen, trifluoromethyl, $C_{1-4}$ alkylsulfonyl, $C_{1-4}$ alkylmercapto, cyano or dimethylamino; Y' is hydrogen, $C_{1-4}$ alkyl, $C_{1-4}$ alkoxy, halogen or it is combined with Y to give 3,4-methylenedioxy; Y'' is hydrogen, $C_{1-4}$ alkyl, $C_{1-4}$ alkoxy or halogen; with R and R' being selected in such a way that at least one of them is other than hydrogen; or the pharmaceutically acceptable salts of the aforesaid compounds, which comprises reacting castanospermine with the appropriate acid halide or anhydride in an inert solvent.

**6.** A process according to Claim 1 for preparing a compound of the formula

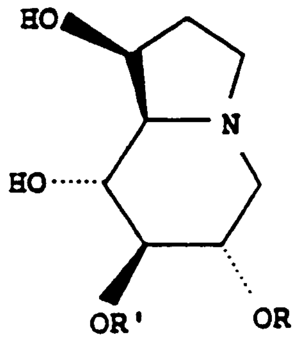

wherein R and R' are independently hydrogen or

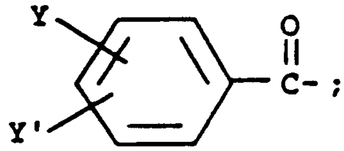

Y is hydrogen, $C_{1-4}$ alkyl, $C_{1-4}$ alkoxy, halogen, trifluoromethyl, $C_{1-4}$ alkylsulfonyl, $C_{1-4}$ alkylmercapto, cyano or dimethylamino; Y' is hydrogen, $C_{1-4}$ alkyl, $C_{1-4}$ alkoxy, halogen or it is combined with Y to give 3,4-methylenedioxy; with R and R' being selected in such a way that at least one of them is other than hydrogen; or the pharmaceutically acceptable salts of the aforesaid compounds, which comprises reacting castanospermine with the appropriate acid halide or anhydride in an inert solvent.

**7.** A process according to Claim 1 for preparing a compound of the formula

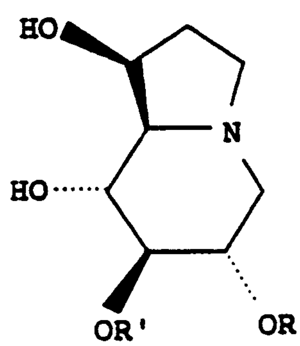

wherein R and R′ are independently hydrogen or

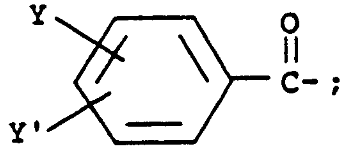

Y is hydrogen or halogen; Y′ is hydrogen or halogen; with R and R′ being selected in such a way that at least one of them is other than hydrogen; or the pharmaceutically acceptable salts of the aforesaid compounds, which comprises reacting castanospermine with the appropriate acid halide or anhydride in an inert solvent.

**8.** A process according to Claim 1 for preparing [1S-(1α,6β,7α,8β,8aβ)]-octahydro-1,6,7,8-indolizinetetrol 6-benzoate which comprises reacting castanospermine with benzoyl chloride.

**9.** A process according to Claim 1 for preparing [1S-(1α,6β,7α,8β,8aβ)]-octahydro-1,6,7,8-indolizinetetrol 7-benzoate which comprises reacting castanospermine with benzoyl chloride.

**10.** A process according to Claim 1 for preparing [1S-(1α,6β,7α,8β,8aβ)]-octahydro-1,6,7,8-indolizinetetrol 6-(4-fluorobenzoate) which comprises reacting castanospermine with 4-fluorobenzoyl chloride.

**11.** A process according to Claim 1 for preparing [1S-(1α,6β,7α,8β,8aβ)]-octahydro-1,6,7,8-indolizinetetrol 7-(4-fluorobenzoate) which comprises reacting castanospermine with 4-fluorobenzoyl chloride.

**12.** A process according to Claim 1 for preparing [1S-(1α,6β,7α,8β,8aβ)]-octahydro-1,6,7,8-indolizinetetrol 6,8-dibutanoate which comprises reacting castanospermine with butyric anhydride.

**13.** A process according to claim 1 for preparing [1S-(1α, 6β,7α, 8β, 8αβ)]-octahydro-1,6,7,8-indolizinetetrol 6-butanoate which comprises reacting castanospermine with butyryl chloride.

**14.** A process according to Claim 1 for preparing a compound of the formula

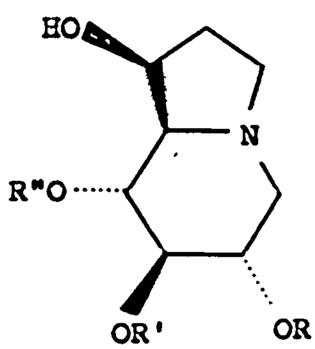

wherein R, R' and R'' are independently hydrogen, or a glycosyl, an *O*-methylglycosyl or Ac-acylated glycosyl radical containing from 1 to 3 hexose or pentose units with attachment at the 1-position of the glycosyl radical; Ac is benzoyl or $C_{2-6}$ alkanoyl; with R, R' and R'' being selected in such a way that at least one of them, but not more than two of them, is hydrogen; or the pharmaceutically acceptable salts of the aforesaid compounds, which comprises reacting castanospermine or an esterified castanospermine with the appropriate Ac-acylated or *O*-benzylated glycosyl halide or acetimidate in an inert solvent followed by catalytic hydrogenation to remove the benzyl groups or optionally by treatment with base to remove the Ac-groups or other ester groups.

**15.** A process according to Claim 1 for preparing 8-*O*-(α-*D*-glucopyranosyl)-[1S-(1α,6β,7α,8β,8aβ) )-octahydro-1,6,7,8-indolizinetetrol which comprises reacting [1S-(1α,6β,7α,8β,8aβ)]-octahydro-1,6,7,8-indolizinetetrol 6,7-dibenzoate with 2,3,4,6-tetra-*O*-(phenylmethyl)-α-*D*-glucopyranosyl trichloroacetimidate followed by treatment with base and then hydrogenation over palladium on charcoal.

**Patentansprüche**
**Patentansprüche für folgende Vertragsstaaten : AT, BE, CH, DE, FR, GB, IT, LI, LU, NL, SE**

1. Verbindung der Formel:

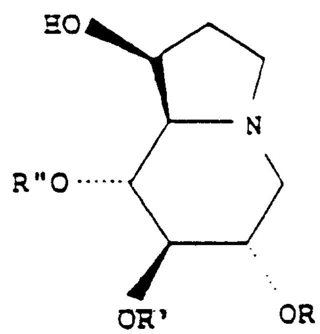

in der R, R' und R" unabhängig voneinander ein Wasserstoffatom, einen $C_{1-10}$-Alkanoylrest, eine Cyclohexancarbonylgruppe, einen Rest der Formel

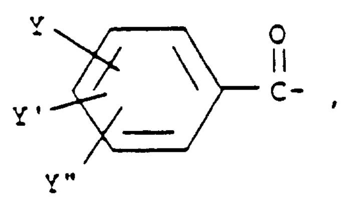

eine gegebenfalls methyl- oder halogen-substituierte Naphthalincarbonylgruppe; einen Phenyl($C_{2-6}$-alkanoyl)rest, in dem die Phenylgruppe gegebenfalls methyl- oder halogen-substituiert ist; eine Cinnamoylgruppe; eine gegebenenfalls methyl- oder halogen-substituierte Pyridincarbonylgruppe; eine gegebenenfalls methyl-substituierte Thiophencarbonylgruppe; eine gegebenenfalls methyl-substituierte Furancarbonylgruppe; oder einen Glykosyl-, *O*-Methylglykosyl- oder Ac-acetylierten Glykosylrest mit 1 bis 3 Hexose- oder Pentoseeinheiten, die in der 1-Stellung des Glykosylrestes gebunden sind, bedeuten; Y ein Wasserstoffatom, $C_{1-4}$-Alkyl-, $C_{1-4}$-Alkoxyrest, Halogenatom, eine Trifluormethylgruppe, ein $C_{1-4}$-Alkylsulfonyl-, $C_{1-4}$-Alkylmercaptorest, eine Cyano- oder Dimethylaminogruppe ist; Y' ein Wasserstoffatom, einen $C_{1-4}$-Alkyl-, $C_{1-4}$-Alkoxyrest, ein Halogenatom bedeutet oder mit Y so kombiniert ist, daß sich eine 3,4-Methylendioxygruppe ergibt; Y" ein Wasserstoffatom, einen $C_{1-4}$-Alkyl-, $C_{1-4}$-Alkoxyrest oder ein Halogenatom darstellt; Ac eine Benzoylgruppe oder ein $C_{2-6}$-Alkanoylrest ist; wobei R, R' und R" so ausgewählt sind, daß wenigstens einer von ihnen, jedoch nicht mehr als zwei von ihnen ein Wasserstoffatom sind; oder die pharmazeutisch verträglichen Salze der vorstehenden Verbindungen.

2. Verbindung nach Anspruch 1 der Formel:

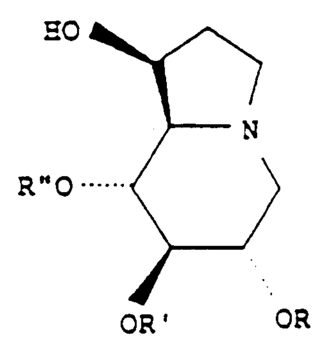

in der R, R' und R" unabhängig voneinander ein Wasserstoffatom, einen $C_{1-10}$-Alkanoylrest, eine Cyclohexancarbonylgruppe, einen Rest der Formel

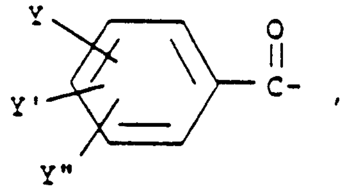

eine gegebenfalls methyl- oder halogen-substituierte Naphthalincarbonylgruppe; einen Phenyl($C_{2-6}$-alkanoyl)rest, in dem die Phenylgruppe gegebenfalls methyl- oder halogen-substituiert ist; eine Cinnamoylgruppe; eine gegebenenfalls methyl- oder halogen-substituierte Pyridincarbonylgruppe; eine gegebenenfalls methyl-substituierte Thiophencarbonylgruppe; eine gegebenenfalls methyl-substituierte Furancarbonylgruppe bedeuten; Y ein Wasserstoffatom, $C_{1-4}$-Alkyl-, $C_{1-4}$-Alkoxyrest, Halogenatom, eine Trifluormethylgruppe, ein $C_{1-4}$-Alkylsulfonyl-, $C_{1-4}$-Alkylmercaptorest, eine Cyano- oder Dimethylaminogruppe ist; Y' ein Wasserstoffatom, einen $C_{1-4}$-Alkyl-,$C_{1-4}$-Alkoxyrest, ein Halogenatom bedeutet oder mit Y so kombiniert ist, daß sich eine 3,4-Methylendioxygruppe ergibt; Y" ein Wasserstoffatom, einen $C_{1-4}$-Alkyl-, $C_{1-4}$-Alkoxyrest oder ein Halogenatom darstellt; wobei R, R' und R" so ausgewählt sind, daß wenigstens einer von ihnen, jedoch nicht mehr als zwei von ihnen ein Wasserstoffatom sind; oder die pharmazeutisch verträglichen Salze der vorstehenden Verbindungen.

**3.** Verbindung nach Anspruch 1 der Formel:

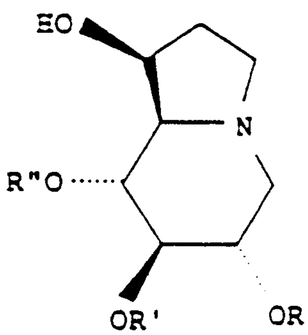

in der R, R' und R" unabhängig voneinander ein Wasserstoffatom, einen $C_{1-10}$-Alkanoylrest oder einen Rest der Formel

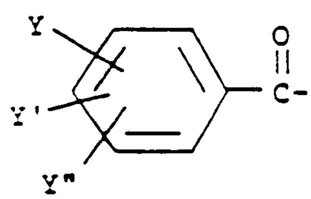

bedeuten; Y ein Wasserstoffatom, $C_{1-4}$-Alkyl-, $C_{1-4}$-Alkoxyrest, Halogenatom, eine Trifluormethylgruppe, ein $C_{1-4}$-Alkylsulfonyl-, $C_{1-4}$-Alkylmercaptorest, eine Cyano- oder Dimethylaminogruppe ist; Y' ein Wasserstoffatom, einen $C_{1-4}$-Alkyl-, $C_{1-4}$-Alkoxyrest, ein Halogenatom bedeutet oder mit Y so kombiniert ist, daß sich eine 3,4-Methylendioxygruppe ergibt; Y" ein Wasserstoffatom, einen $C_{1-4}$-Alkyl-, $C_{1-4}$-Alkoxyrest oder ein Halogenatom darstellt; wobei R, R' und R" so ausgewählt sind, daß wenigstens einer von ihnen, jedoch nicht mehr als zwei von ihnen ein Wasserstoffatom sind; oder die pharmazeutisch verträglichen Salze der vorstehenden Verbindungen.

**4.** Verbindung nach Anspruch 1 der Formel:

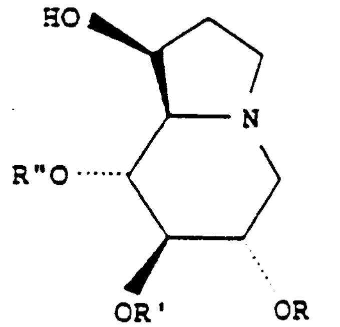

in der R, R' und R'' unabhängig voneinander ein Wasserstoffatom oder einen Rest der Formel

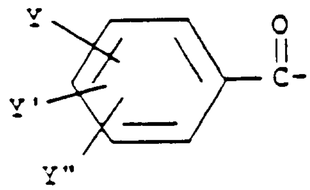

bedeuten; Y ein Wasserstoffatom, $C_{1-4}$-Alkyl-, $C_{1-4}$-Alkoxyrest, Halogenatom, eine Trifluormethylgruppe, ein $C_{1-4}$-Alkylsulfonyl-, $C_{1-4}$-Alkylmercaptorest, eine Cyano- oder Dimethylaminogruppe ist; Y' ein Wasserstoffatom, einen $C_{1-4}$-Alkyl-, $C_{1-4}$-Alkoxyrest, ein Halogenatom bedeutet oder mit Y so kombiniert ist, daß sich eine 3,4-Methylendioxygruppe ergibt; Y'' ein Wasserstoffatom, einen $C_{1-4}$-Alkyl-, $C_{1-4}$-Alkoxyrest oder ein Halogenatom darstellt; wobei R, R' und R'' so ausgewählt sind, daß wenigstens einer von ihnen, jedoch nicht mehr als zwei von ihnen ein Wasserstoffatom sind; oder die pharmazeutisch verträglichen Salze der vorstehenden Verbindungen.

**5.** Verbindung nach Anspruch 1 der Formel:

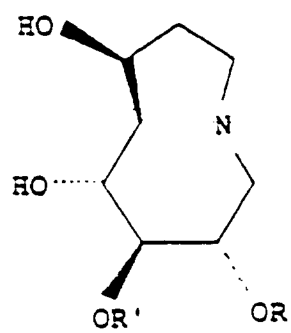

in der R und R' unabhängig voneinander ein Wasserstoffatom oder einen Rest der Formel

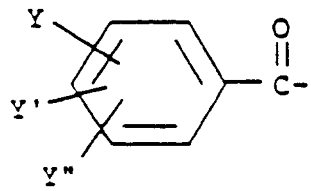

bedeuten; Y ein Wasserstoffatom, $C_{1-4}$-Alkyl-, $C_{1-4}$-Alkoxyrest, Halogenatom, eine Trifluormethylgruppe, ein $C_{1-4}$-Alkylsulfonyl-, $C_{1-4}$-Alkylmercaptorest, eine Cyano- oder Dimethylaminogruppe ist; Y' ein Wasserstoffatom, einen $C_{1-4}$-Alkyl-, $C_{1-4}$-Alkoxyrest, ein Halogenatom bedeutet oder mit Y so kombiniert ist, daß sich eine 3,4-Methylendioxygruppe ergibt; Y'' ein Wasserstoffatom, einen $C_{1-4}$-Alkyl-, $C_{1-4}$-Alkoxyrest oder ein Halogenatom darstellt; wobei R und R' so ausgewählt sind, daß wenigstens einer von ihnen kein Wasserstoffatom ist; oder die pharmazeutisch verträglichen Salze der vorstehenden Verbindungen.

6. Verbindung nach Anspruch 1 der Formel:

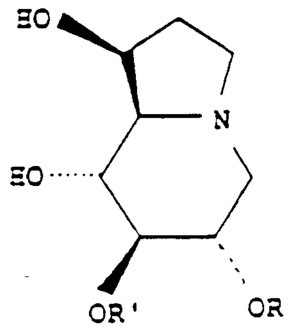

in der R und R' unabhängig voneinander ein Wasserstoffatom oder einen Rest der Formel

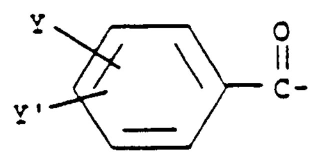

bedeuten; Y ein Wasserstoffatom, $C_{1-4}$-Alkyl-, $C_{1-4}$-Alkoxyrest, Halogenatom, eine Trifluormethylgruppe, ein $C_{1-4}$-Alkylsulfonyl-, $C_{1-4}$-Alkylmercaptorest, eine Cyano- oder Dimethylaminogruppe ist; Y' ein Wasserstoffatom, einen $C_{1-4}$-Alkyl-, $C_{1-4}$-Alkoxyrest, ein Halogenatom bedeutet oder mit Y so kombiniert ist, daß sich eine 3,4-Methylendioxygruppe ergibt; wobei R und R' so ausgewählt sind, daß wenigstens einer von ihnen kein Wasserstoffatom ist; oder die pharmazeutisch verträglichen Salze der vorstehenden Verbindungen.

7. Verbindung nach Anspruch 1 der Formel:

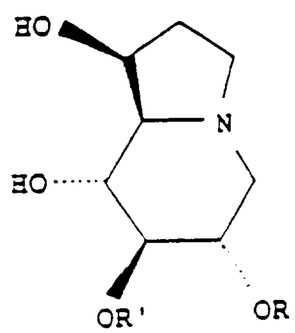

in der R und R' unabhängig voneinander ein Wasserstoffatom oder einen Rest der Formel

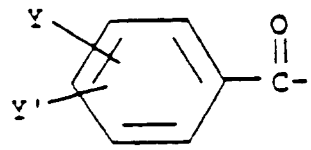

bedeuten; Y ein Wasserstoff- oder Halogenatom ist; Y' ein Wasserstoff- oder Halogenatom darstellt, wobei R und R' so ausgewählt sind, daß wenigstens einer von ihnen kein Wasserstoffatom ist; oder die pharmazeutisch verträglichen Salze der vorstehenden Verbindungen.

**8.** Verbindung nach Anspruch 1, nämlich [1S-(1α,6β,7α,8β,8aβ)]-Octahydro-1,6,7,8-indolizintetrol-6-benzoat.

**9.** Verbindung nach Anspruch 1, nämlich [1S-(1α,6β,7α,8β,8aβ)]-Octahydro-1,6,7,8-indolizintetrol-7-benzoat.

**10.** Verbindung nach Anspruch 1, nämlich [1S-(1α,6β,7α,8β,8aβ)]-Octahydro-1,6,7,8-indolizintetrol-6-(4-fluorbenzoat).

**11.** Verbindung nach Anspruch 1, nämlich [1S-(1α,6β,7α,8β,8aβ)]-Octahydro-1,6,7,8-indolizintetrol-7(4-fluorbenzoat).

**12.** Verbindung nach Anspruch 1, nämlich [1S-(1α,6β,7α,8β,8aβ)]-Octahydro-1,6,7,8-indolizintetrol-6,8-dibutanoat.

**13.** Verbindung nach Anspruch 1, nämlich [1S-(1α,6β,7α,8β,8aβ)]-Octahydro-1,6,7,8-indolizintetrol-6-butanoat.

**14.** Verbindung nach Anspruch 1 der Formel:

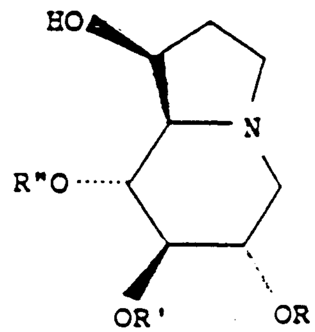

in der R, R' und R" unabhängig voneinander ein Wasserstoffatom oder einen Glykosyl-, *O*-Methylglykosyl- oder Ac-acetylierten Glykosylrest mit 1 bis 3 Hexose- oder Pentoseeinheiten, die in der 1-Stellung des Glykosylrestes gebunden sind, bedeuten; Ac eine Benzoylgruppe oder ein $C_{2-6}$-Alkanoylrest ist; wobei R, R' und R" so ausgewählt sind, daß wenigstens einer von ihnen, jedoch nicht mehr als zwei von ihnen ein Wasserstoffatom sind; oder die pharmazeutisch verträglichen Salze der vorstehenden Verbindungen.

**15.** Verbindung nach Anspruch 1, nämlich 8-*O*-(α-*D*-Glucopyranosyl)-[1S-(1α,6β,7α,8β,8aβ)]-octahydro-1,6,7,8-indolizintetrol.

**16.** Verfahren zur Herstellung einer Verbindung nach Anspruch 1, das die Umsetzung von Castanospermin mit entweder:

(a) dem geeigneten Säurehalogenid oder -anhydrid in einem inerten Lösungsmittel, oder

(b) dem geeigneten Ac-acylierten oder *O*-benzylierten Glykosylhalogenid oder -acetimidat in einem inerten Lösungsmittel und die anschließende katalytische Hydrierung zum Entfernen der Benzylgruppen oder gegebenenfalls die Behandlung mit einer Base zum Entfernen der Ac-Gruppen oder anderer Estergruppen

umfaßt.

**17.** Verbindung nach Anspruch 1 zur Verwendung als Arzneimittel.

**18.** Verbindung nach Anspruch 2 zur Verwendung als Arzneimittel.

**19.** Verbindung nach einem der Ansprüche 17 und 18 zur Verwendung bei der Behandlung von Diabetes in Säugern.

**20.** Verbindung nach einem der Ansprüche 17 und 18 zur Verwendung bei der Behandlung von postprandialer Hyperglykämie bei Individuen mit Diabetes.

**21.** Verbindung nach Anspruch 1 zur Verwendung bei Herstellung eines Arzneimittels für die Behandlung von Diabetes in Säugern oder von postprandialer Hyperglykämie bei Individuen mit Diabetes.

**22.** Verbindung nach Anspruch 2 zur Verwendung bei Herstellung eines Arzneimittels für die Behandlung von Diabetes in Säugern oder von postprandialer Hyperglykämie bei Individuen mit Diabetes.

**23.** Pharmazeutische Dosierungsform, die eine Verbindung nach Anspruch 1 enthält.

**24.** Pharmazeutische Dosierungsform, die eine Verbindung nach Anspruch 2 enthält.

**Patentansprüche für folgende Vertragsstaaten : ES, GR**

**1.** Verfahren zur Herstellung einer Verbindung der Formel:

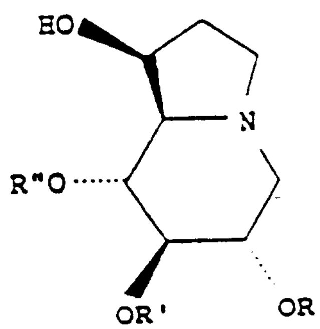

in der R, R' und R" unabhängig voneinander ein Wasserstoffatom, einen $C_{1-10}$-Alkanoylrest, eine Cyclohexancarbonylgruppe, einen Rest der Formel

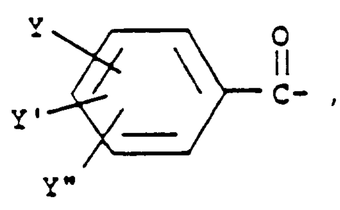

eine gegebenfalls methyl- oder halogen-substituierte Naphthalincarbonylgruppe; einen Phenyl($C_{2-6}$-alkanoyl)rest, in dem die Phenylgruppe gegebenfalls methyl- oder halogen-substituiert ist; eine Cinnamoylgruppe; eine gegebenenfalls methyl- oder halogen-substituierte Pyridincarbonylgruppe; eine gegebenenfalls methyl-substituierte Thiophencarbonylgruppe; eine gegebenenfalls methyl-substituierte Furancarbonylgruppe; oder einen Glykosyl-, *O*-Methylglykosyl- oder Ac-acetylierten Glykosylrest mit 1 bis 3 Hexose- oder Pentoseeinheiten, die in der 1-Stellung des Glykosylrestes gebunden sind, bedeuten; Y ein Wasserstoffatom, $C_{1-4}$-Alkyl-, $C_{1-4}$-Alkoxyrest, Halogenatom, eine Trifluormethylgruppe, ein $C_{1-4}$-Alkylsulfonyl-, $C_{1-4}$-Alkylmercaptorest, eine Cyano- oder Dimethylaminogruppe ist; Y' ein Wasserstoffatom, einen $C_{1-4}$-Alkyl-, $c_{1-4}$-Alkoxyrest, ein Halogenatom bedeutet oder mit Y so kombiniert ist, daß sich eine 3,4-Methylendioxygruppe ergibt; Y" ein Wasserstoffatom, einen $C_{1-4}$-Alkyl-, $C_{1-4}$-Alkoxyrest oder ein Halogenatom darstellt; Ac eine Benzoylgruppe oder ein $C_{2-6}$-Alkanoylrest ist; wobei R, R' und R" so ausgewählt sind, daß wenigstens einer von ihnen, jedoch nicht mehr als zwei von ihnen ein Wasserstoffatom sind; oder der pharmazeutisch verträglichen Salze der vorstehenden Verbindungen, das die Umsetzung von Castanospermin mit entweder:

(a) dem geeigneten Säurehalogenid oder -anhydrid in einem inerten Lösungsmittel; oder

(b) dem geeigneten Ac-acylierten oder *O*-benzylierten Glykosylhalogenid oder -acetimidat in einem inerten Lösungsmittel und die anschließende katalytische Hydrierung zum Entfernen der Benzylgruppen oder gegebenenfalls die Behandlung mit einer Base zum Entfernen der Ac-Gruppen oder anderer Estergruppen

umfaßt.

**2.** Verfahren nach Anspruch 1 zur Herstellung einer Verbindung der Formel:

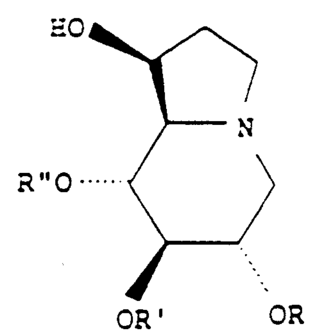

in der R, R' und R" unabhängig voneinander ein Wasserstoffatom, einen $C_{1-10}$-Alkanoylrest, eine Cyclohexancarbonylgruppe, einen Rest der Formel

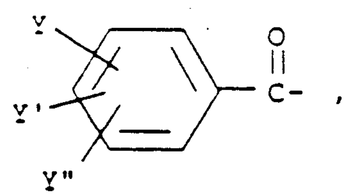

eine gegebenfalls methyl- oder halogen-substituierte Naphthalincarbonylgruppe; einen Phenyl($C_{2-6}$-alkanoyl)rest, in dem die Phenylgruppe gegebenfalls methyl- oder halogen-substituiert ist; eine Cinnamoylgruppe; eine gegebenenfalls methyl- oder halogen-substituierte Pyridincarbonylgruppe; eine gegebenenfalls methyl-substituierte Thiophencarbonylgruppe; eine gegebenenfalls methyl-substituierte Furancarbonylgruppe bedeuten; Y ein Wasserstoffatom, $c_{1-4}$-Alkyl-, $C_{1-4}$-Alkoxyrest, Halogenatom, eine Trifluormethylgruppe, ein $C_{1-4}$-Alkylsulfonyl-, $C_{1-4}$-Alkylmercaptorest, eine Cyano- oder Dimethylaminogruppe ist; Y' ein Wasserstoffatom, einen $C_{1-4}$-Alkyl-,$C_{1-4}$-Alkoxyrest, ein Halogenatom bedeutet oder mit Y so kombiniert ist, daß sich eine 3,4-Methylendioxygruppe ergibt; Y" ein Wasserstoffatom, einen $C_{1-4}$-Alkyl-, $C_{1-4}$-Alkoxyrest oder ein Halogenatom darstellt; wobei R, R' und R" so ausgewählt sind, daß wenigstens einer von ihnen, jedoch nicht mehr als zwei von ihnen ein Wasserstoffatom sind; oder der pharmazeutisch verträglichen Salze der vorstehenden Verbindungen, das die Umsetzung von Castanospermin mit dem geeigneten Säurehalogenid oder -anhydrid in einem inerten Lösungsmittel umfaßt.

**3.** Verfahren nach Anspruch 1 zur Herstellung einer Verbindung der Formel:

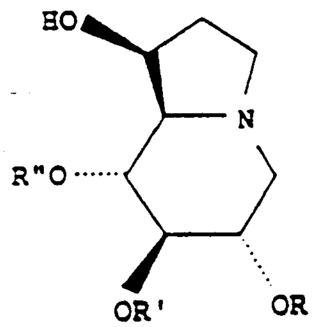

in der R, R' und R'' unabhängig voneinander ein Wasserstoffatom, einen $C_{1-10}$-Alkanoylrest oder einen Rest der Formel

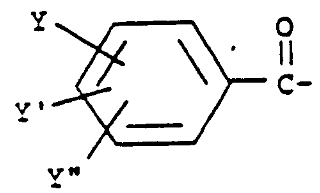

bedeuten; Y ein Wasserstoffatom, $C_{1-4}$-Alkyl-, $C_{1-4}$-Alkoxyrest, Halogenatom, eine Trifluormethylgruppe, ein $C_{1-4}$-Alkylsulfonyl-, $C_{1-4}$-Alkylmercaptorest, eine Cyano- oder Dimethylaminogruppe ist; Y' ein Wasserstoffatom, einen $C_{1-4}$-Alkyl-, $C_{1-4}$-Alkoxyrest, ein Halogenatom bedeutet oder mit Y so kombiniert ist, daß sich eine 3,4-Methylendioxygruppe ergibt; Y'' ein Wasserstoffatom, einen $C_{1-4}$-Alkyl-, $C_{1-4}$-Alkoxyrest oder ein Halogenatom darstellt; wobei R, R' und R'' so ausgewählt sind, daß wenigstens einer von ihnen, jedoch nicht mehr als zwei von ihnen ein Wasserstoffatom sind; oder der pharmazeutisch verträglichen Salze der vorstehenden Verbindungen, das die Umsetzung von Castanospermin mit dem geeigneten Säurehalogenid oder -anhydrid in einem inerten Lösungsmittel umfaßt.

**4.** Verfahren nach Anspruch 1 zur Herstellung einer Verbindung der Formel:

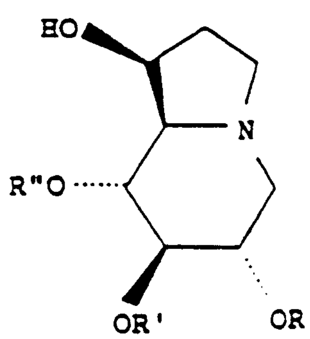

in der R, R' und R'' unabhängig voneinander ein Wasserstoffatom oder einen Rest der Formel

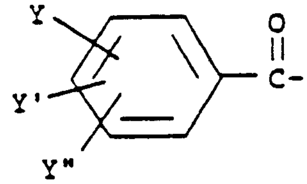

bedeuten; Y ein Wasserstoffatom, $C_{1-4}$-Alkyl-, $C_{1-4}$-Alkoxyrest, Halogenatom, eine Trifluormethylgruppe, ein $C_{1-4}$-Alkylsulfonyl-, $C_{1-4}$-Alkylmercaptorest, eine Cyano- oder Dimethylaminogruppe ist; Y' ein Wasserstoffatom, einen $C_{1-4}$-Alkyl-, $C_{1-4}$-Alkoxyrest, ein Halogenatom bedeutet oder mit Y so kombiniert ist, daß sich eine 3,4-Methylendioxygruppe ergibt; Y'' ein Wasserstoffatom, einen $C_{1-4}$-Alkyl-, $C_{1-4}$-Alkoxyrest oder ein Halogenatom darstellt; wobei R, R' und R'' so ausgewählt sind, daß wenigstens einer von ihnen, jedoch nicht mehr als zwei von ihnen ein Wasserstoffatom sind; oder der pharmazeutisch verträglichen Salze der vorstehenden Verbindungen, das die Umsetzung von Castanospermin mit dem geeigneten Säurehalogenid oder -anhydrid in einem inerten Lösungsmittel umfaßt.

**5.** Verfahren nach Anspruch 1 zur Herstellung einer Verbindung der Formel:

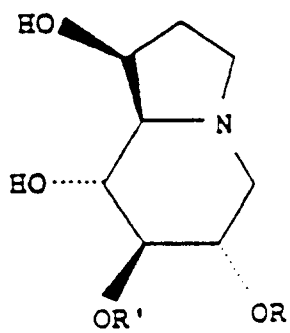

in der R und R' unabhängig voneinander ein Wasserstoffatom oder einen Rest der Formel

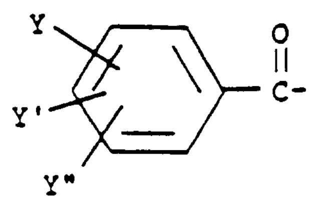

bedeuten; Y ein Wasserstoffatom, $C_{1-4}$-Alkyl-, $C_{1-4}$-Alkoxyrest, Halogenatom, eine Trifluormethylgruppe, ein $C_{1-4}$-Alkylsulfonyl-, $C_{1-4}$-Alkylmercaptorest, eine Cyano- oder Dimethylaminogruppe ist; Y' ein Wasserstoffatom, einen $C_{1-4}$-Alkyl-, $C_{1-4}$-Alkoxyrest, ein Halogenatom bedeutet oder mit Y so kombiniert ist, daß sich eine 3,4-Methylendioxygruppe ergibt; Y'' ein Wasserstoffatom, einen $C_{1-4}$-Alkyl-, $C_{1-4}$-Alkoxyrest oder ein Halogenatom darstellt; wobei R und R' so ausgewählt sind, daß wenigstens einer von ihnen kein Wasserstoffatom ist; oder der pharmazeutisch verträglichen Salze der vorstehenden Verbindungen, das die Umsetzung von Castanospermin mit dem geeigneten Säurehalogenid oder -anhydrid in einem inerten Lösungsmittel umfaßt.

6. Verfahren nach Anspruch 1 zur Herstellung einer Verbindung der Formel:

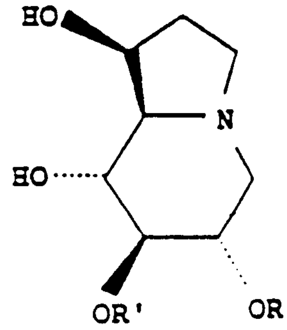

in der R und R' unabhängig voneinander ein Wasserstoffatom oder einen Rest der Formel

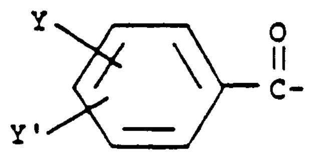

bedeuten; Y ein Wasserstoffatom, $C_{1-4}$-Alkyl-, $C_{1-4}$-Alkoxyrest, Halogenatom, eine Trifluormethylgruppe, ein $C_{1-4}$-Alkylsulfonyl-, $C_{1-4}$-Alkylmercaptorest, eine Cyano- oder Dimethylaminogruppe ist; Y' ein Wasserstoffatom, einen $C_{1-4}$-Alkyl-, $C_{1-4}$-Alkoxyrest, ein Halogenatom bedeutet oder mit Y so kombiniert ist, daß sich eine 3,4-Methylendioxygruppe ergibt; wobei R und R' so ausgewählt sind, daß wenigstens einer von ihnen kein Wasserstoffatom ist; oder der pharmazeutisch verträglichen Salze der vorstehenden Verbindungen, das die Umsetzung von Castanospermin mit dem geeigneten Säurehalogenid oder -anhydrid in einem inerten Lösungsmittel umfaßt.

7. Verfahren nach Anspruch 1 zur Herstellung einer Verbindung der Formel:

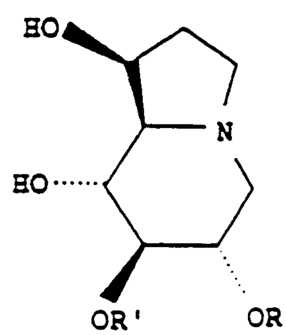

in der R und R' unabhängig voneinander ein Wasserstoffatom oder einen Rest der Formel

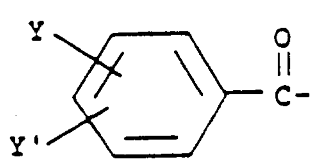

bedeuten; Y ein Wasserstoff- oder Halogenatom ist; Y' ein Wasserstoff- oder Halogenatom darstellt, wobei R und R' so ausgewählt sind, daß wenigstens einer von ihnen kein Wasserstoffatom ist; oder der

pharmazeutisch verträglichen Salze der vorstehenden Verbindungen, das die Umsetzung von Castanospermin mit dem geeigneten Säurehalogenid oder -anhydrid in einem inerten Lösungsmittel umfaßt.

8. Verfahren nach Anspruch 1 zur Herstellung von [1S-(1α,6β,7α,8β,8aβ)]-Octahydro-1,6,7,8-indolizintetrol-6-benzoat, das die Umsetzung von Castanospermin mit Benzoylchlorid umfaßt.

9. Verfahren nach Anspruch 1 zur Herstellung von [1S-(1α,6β,7α,8β,8aβ)]-Octahydro-1,6,7,8-indolizintetrol-7-benzoat, das die Umsetzung von Castanospermin mit Benzoylchlorid umfaßt.

10. Verfahren nach Anspruch 1 zur Herstellung von [1S-(1α,6β,7α,8β,8aβ)]-Octahydro-1,6,7,8-indolizintetrol-6-(4-fluorbenzoat), das die Umsetzung von Castanospermin mit 4-Fluorbenzoylchlorid umfaßt.

11. Verfahren nach Anspruch 1 zur Herstellung von [1S-(1α,6β,7α,8β,8aβ)]-Octahydro-1,6,7,8-indolizintetrol-7-(4-fluorbenzoat), das die Umsetzung von Castanospermin mit 4-Fluorbenzoylchlorid umfaßt.

12. Verfahren nach Anspruch 1 zur Herstellung von [1S-(1α,6β,7α,8β,8aβ)]-Octahydro-1,6,7,8-indolizintetrol-6,8-dibutanoat, das die Umsetzung von Castanospermin mit Buttersäureanhydrid umfaßt.

13. Verfahren nach Anspruch 1 zur Herstellung von [1S-(1α,6β,7α,8β,8aβ)]-Octahydro-1,6,7,8-indolizintetrol-6-butanoat, das die Umsetzung von Castanospermin mit Buttersäurechlorid umfaßt.

14. Verfahren nach Anspruch 1 zur Herstellung einer Verbindung der Formel:

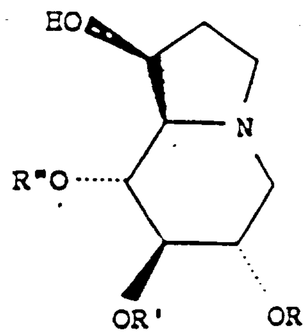

in der R, R' und R'' unabhängig voneinander ein Wasserstoffatom oder einen Glykosyl-, *O*-Methylglykosyl- oder Ac-acetylierten Glykosylrest mit 1 bis 3 Hexose- oder Pentoseeinheiten, die in der 1-Stellung des Glykosylrestes gebunden sind, bedeuten; Ac eine Benzoylgruppe oder ein $C_{2-6}$-Alkanoylrest ist; wobei R, R' und R'' so ausgewählt sind, daß wenigstens einer von ihnen, jedoch nicht mehr als zwei von ihnen ein Wasserstoffatom sind; oder der pharmazeutisch verträglichen Salze der vorstehenden Verbindungen, das die Umsetzung von Castanospermin oder einem veresterten Castanospermin mit dem geeigneten Ac-acylierten oder *O*-benzylierten Glykosylhalogenid oder -acetimidat in einem inerten Lösungsmittel und die anschließende katalytische Hydrierung zum Entfernen der Benzylgruppen oder gegebenenfalls die Behandlung mit einer Base zum Entfernen der Ac-Gruppen oder anderer Estergruppen umfaßt.

15. Verfahren nach Anspruch 1 zur Herstellung von 8-*O*-(α-*D*-Glucopyranosyl)-[1S-(1α,6β,7α,8β,8aβ)]-octahydro-1,6,7,8-indolizintetrol, das die Umsetzung von [1S-(1α,6β,7α,8β,8aβ)]-Octahydro-1,6,7,8-indolizintetrol-6,7-dibenzoat mit 2,3,4,6-Tetra-*O*-(phenylmethyl)-α-*D*-glucopyranosyltrichloracetimidat und die anschließende Behandlung mit einer Base und dann die Hydrierung über Palladium auf Holzkohle umfaßt.

**Revendications**

**Revendications pour les Etats contractants suivants : AT, BE, CH, DE, FR, GB, IT, LI, LU, NL, SE**

**1.** Composé de formule :

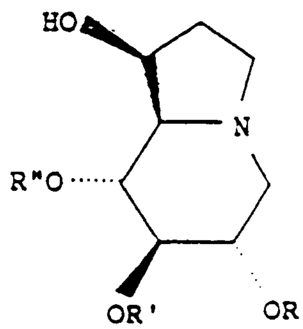

dans laquelle R, R' et R" représentent chacun, indépendamment l'un de l'autre, un atome d'hydrogène, un groupe alcanoyle en $C_1$-$C_{10}$, un groupe cyclohexane carbonyle,

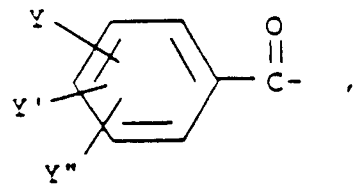

un groupe naphtalènecarbonyle éventuellement substitué par un reste méthyle ou halogéno ; un groupe phényl-(alcanoyle en $C_2$-$C_6$), dans lequel le noyau phényle est éventuellement substitué par un reste méthyle ou halogéno ; un groupe cinnamoyle ; un groupe pyridinecarbonyle éventuellement substitué par un reste méthyle ou halogéno; un groupe thiophènecarbonyle éventuellement substitué par un reste méthyle ; un groupe furannecarbonyle éventuellement substitué par un reste méthyle ; ou un radical glycosyle, un radical O-méthylglycosyle ou glycosyle acylé par un reste Ac contenant 1 à 3 hexoses ou pentoses avec fixation en position 1 du radical glycosyle ; Y représente un atome d'hydrogène, un groupe alkyle en $C_1$-$C_4$, alcoxy en $C_1$-$C_4$, halogéno, trifluorométhyle, alkylsulfonyle en $C_1$-$C_4$, alkylmercapto en $C_1$-$C_4$, cyano ou diméthylamino ; Y' représente un atome d'hydrogène, un groupe alkyle en $C_1$-$C_4$, alcoxy en $C_1$-$C_4$, halogéno, ou bien il est combiné avec Y pour donner un groupe 3,4-méthylènedioxy ; Y" représente un atome d'hydrogène, un groupe alkyle en $C_1$-$C_4$, alcoxy en $C_1$-$C_4$ ou halogéno; Ac représente un groupe benzoyle ou alcanoyle en $c_2$-$c_6$; les sens des symboles R, R' et R" étant choisis de façon qu'au moins l'un de ces symboles, mais pas plus de deux d'entre eux, représente(nt) un atome d'hydrogène, ou les sels pharmaceutiquement acceptables des composés précités.

**2.** Composé selon la revendication 1, qui a pour formule :

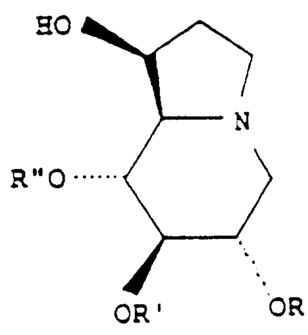

dans laquelle R, R' et R" représentent chacun, indépendamment, un atome d'hydrogène, un groupe

alcanoyle en $C_1$-$C_{10}$, cyclohexanecarbonyle,

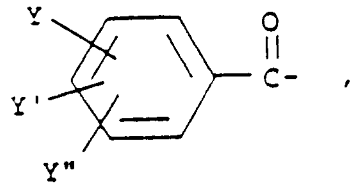

naphtalenecarbonyle éventuellement substitué par un radical méthyle ou halogéno; un groupe phényl-(alcanoyle en $C_2$-$C_6$) dans lequel le noyau phényle est éventuellement substitué par un radical méthyle ou halogéno; un groupe cinnamoyle, pyridinecarbonyle éventuellement substitué par un radical méthyle ou halogéno ; un groupe thiophènecarbonyle éventuellement substitué par un radical méthyle ; un groupe furannecarbonyle éventuellement substitué par un radical méthyle ; Y représente un atome d'hydrogène, un groupe alkyle en $C_1$-$C_4$, alcoxy en $C_1$-$C_4$ halogéno, trifluorométhyle, alkylsulfonyle en $C_1$-$C_4$, alkylmercapto en $C_1$-$C_4$, cyano ou diméthylamino; Y' représente un atome d'hydrogène, un groupe alkyle en $C_1$-$C_4$, alcoxy en $C_1$-$C_4$, halogéno ou bien il est combiné avec Y pour donner un groupe 3,4-méthylènedioxy ; Y" représente un atome d'hydrogène, un groupe alkyle en $C_1$-$C_4$, alcoxy en $C_1$-$C_4$ ou halogéno ; le sens des symboles R, R' et R" étant choisi de façon qu'au moins l'un d'entre eux, mais pas plus de deux d'entre eux, représente(nt) un atome d'hydrogène ; ou les sels pharmaceutiquement acceptables des composés précités.

**3.** Composé selon la revendication 1, qui a pour formule :

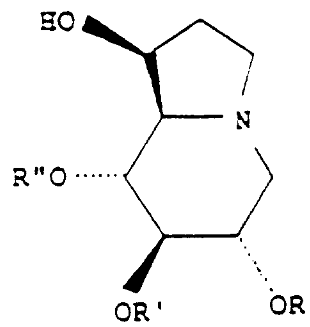

dans laquelle R, R' et R" représentent chacun, indépendamment, un atome d'hy drogène, un groupe alcanoyle en $C_1$-$C_{10}$ ou

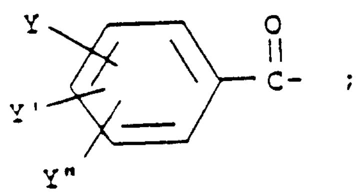

Y représente un atome d'hydrogène, un groupe alkyle en $C_1$-$C_4$, alcoxy en $C_1$-$C_4$, halogéno, trifluorométhyle, alkylsulfonyle en $C_1$-$C_4$, alkylmercapto en $C_1$-$C_4$, cyano ou diméthylamino ; Y' représente un atome d'hydrogène, un groupe alkyle en $C_1$-$C_4$, alcoxy en $C_1$-$C_4$, halogéno ou bien il est combiné avec Y pour donner un groupe 3,4-méthylènedioxy ; Y" représente un atome d'hydrogène, un groupe alkyle en $C_1$-$C_4$, alcoxy en $C_1$-$C_4$ ou halogéno ; le sens des symboles R, R' et R" étant choisis de façon qu'au moins l'un d'entre eux, mais pas plus de deux d'entre eux, représente(nt) un atome d'hydrogène; ou les sels pharmaceutiquement acceptables des composés précités.

**4.** Composé selon la revendication 1, qui a pour formule :

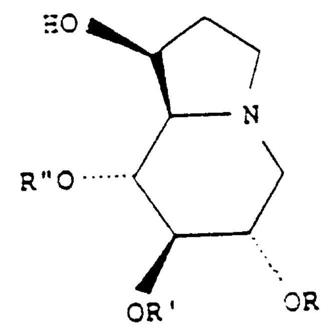

dans laquelle R, R' et R'' representent chacun, indépendamment l'un de l'autre, un atome d'hydrogène ou

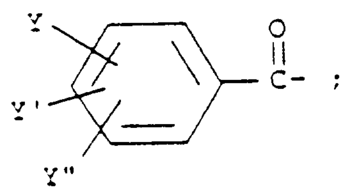

Y représente un atome d'hydrogène, un groupe alkyle en $C_1$-$C_4$, alcoxy en $C_1$-$C_4$, halogéno, trifluorométhyle, alkylsulfonyle en $C_1$-$C_4$, alkylmercapto en $C_1$-$C_4$, cyano ou diméthylamino; Y' représente un atome d'hydrogène, un groupe alkyle en $C_1$-$C_4$, alcoxy en $C_1$-$C_4$, halogéno ou bien il est combiné avec Y pour donner un groupe 3,4-méthylènedioxy ; Y'' représente un atome d'hydrogène, un groupe alkyle en $C_1$-$C_4$, alcoxy en $C_1$-$C_4$ ou halogéno ; le sens des symboles R, R' et R'' étant choisi de façon qu'au moins l'un d'entre eux, mais pas plus de deux d'entre eux, représente(nt) un atome d'hydrogène ou les sels pharmaceutiquement acceptables des composés précités.

**5.** Composé selon la revendication 1, qui a pour formule :

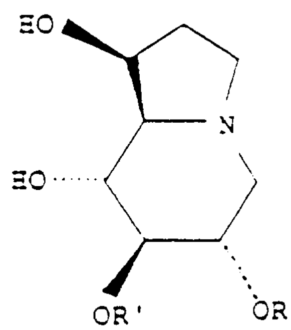

dans laquelle R et R' représentent chacun indépendamment, un atome d'hydrogène ou

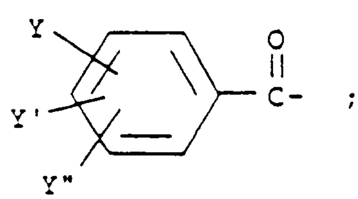

Y représente un atome d'hydrogène, un groupe alkyle en $C_1$-$C_4$, alcoxy en $C_1$-$C_4$, halogéno, trifluoro-

méthyle, alkylsulfonyle en $C_1$-$C_4$, alkylmercapto en $C_1$-$C_4$, cyano ou diméthylamino ; Y' représente un atome d'hydrogène, un groupe alkyle en $C_1$-$C_4$, alcoxy en $C_1$-$C_4$, halogénon ou bien il est combiné avec Y pour former un groupe 3,4-méthylènedioxy ; Y'' représente un atome d'hydrogène, un groupe alkyle en $C_1$-$C_4$, alcoxy en $C_1$-$C_4$ ou halogéno, le sens des symboles R et R' étant choisi de façon qu'au moins l'un d'entre eux représente autre chose qu'un atome d'hydrogène; ou les sels pharmaceutiquement acceptables des composés précités.

6. Composé selon la revendication 1, qui a pour formule :

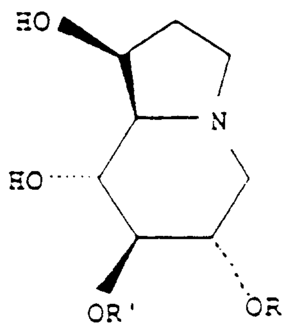

dans laquelle R et R' représentent chacun, indépendamment, un atome d'hydrogène ou

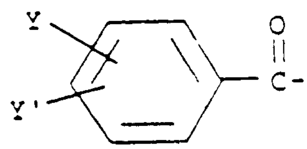

;

Y représente un atome d'hydrogène, un groupe alkyle en $C_1$-$C_4$, alcoxy en $C_1$-$C_4$, halogéno, trifluorométhyle, alkylsulfonyle en $C_1$-$C_4$, alkylmercapto en $C_1$-$C_4$, cyano ou diméthylamino Y' représente un atome d'hydrogène, un groupe alkyle en $C_1$-$C_4$, alcoxy en $C_1$-$C_4$, halogéno ou bien il est combiné avec Y pour donner un groupe 3,4-méthylènedioxy ; le sens des symboles R et R' étant choisi de façon qu'au moins l'un d'entre eux représente autre chose qu'un atome d'hydrogène ; ou les sels pharmaceutiquement acceptables des composés précités.

7. Composé selon la revendication 1, qui a pour formule :

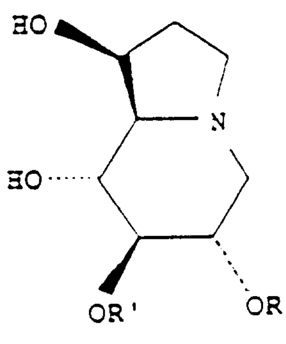

dans laquelle R et R' représentent chacun, indépendamment, un atome d'hydrogène ou

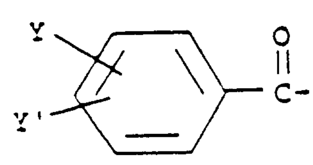

Y représente un atome d'hydrogène ou d'halogène; Y' représente un atome d'hydrogène ou d'halogène; le sens des symboles R et R' étant choisi de façon qu'au moins l'un d'entre eux représente autre chose qu'un atome d'hydrogène ; ou les sels pharmaceutiquement acceptables des composés précités.

**8.** Composé selon la revendication 1, qui est le 6-benzoate de [1S-(1α,6β, 7α, 8β, 8aβ)]-octahydro-1,6,7,8-indolizinetétrol.

**9.** Composé selon la revendication 1, qui est le 7-benzoate de [1S-(1α,6β, 7α, 8β, 8aβ)]-octahydro-1,6,7,8-indolizinetétrol.

**10.** Composé selon la revendication 1, qui est le 6-(4-fluorobenzoate) de [1S-(1α,6β, 7α, 8β, 8aβ)]-octahydro-1,6,7,8-indolizinetétrol.

**11.** Composé selon la revendication 1, qui est le 7-(4-fluorobenzoate) de [1S-(1α,6β, 7α, 8β, 8aβ)]-octahydro-1,6,7,8-indolizinetétrol.

**12.** Composé selon la revendication 1, qui est le 6,8-dibutanoate de [1S-(1α,6β, 7α, 8β, 8aβ)]-octahydro-1,6,7,8-indolizinetétrol.

**13.** Composé selon la revendication 1, qui est le 6-butanoate de [1S-(1α,6β, 7α, 8β, 8aβ)]-octahydro-1,6,7,8-indolizinetétrol.

**14.** Composé selon la revendication 1, qui a pour formule :

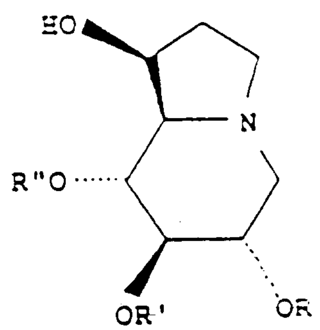

dans laquelle R, R' et R'' représentent chacun, indépendamment l'un de l'autre, un atome d'hydrogène, ou un radical glycosyle, un radical O-méthylglycosyle ou un radical glycosyle acylé Ac et contenant 1 à 3 motifs hexose ou pentose, avec fixation en position 1 du radical glycosyle ; Ac représente un groupe benzoyle ou alcanoyle en $C_2$-$C_6$, le sens des symboles R, R' et R'' étant choisi de façon qu'au moins l'un d'entre eux, mais pas plus de deux d'entre eux, représente(n) un atome d'hydrogène ; ou les sels pharmaceutiquement acceptables des composés précités.

**15.** Composé selon la revendication 1, qui est le 8-O-(α-D-glucopyrannosyl)-[1S-(1α,6β, 7α, 8β, 8aβ)]-octahydro-1,6,7,8-indolizinetétrol.

**16.** Procédé pour préparer un composé selon la revendication 1, qui comprend la réaction de la castanospermine avec :

(a) l'halogènure ou anhydride d'acide approprié dans un solvant inerte; ou bien

(b) avec l'halogénure ou acétimidate de glycosyle acylé par Ac u O-benzylaté, dans un solvant inerte, opération suivie d'une hydrogénation catalytique pour enlever les groupes benzyle ou éventuellement un traitement par une base pour enlever les groupes Ac ou autres groupes esters.

**17.** Composé selon la revendication 1, destiné à servir de médicament.

**18.** Composé selon la revendication 2, destiné à servir de médicament.

**19.** Composé selon l'une quelconque des revendications 17 et 18, destiné à servir au traitement du diabète chez des mammifères.

**20.** Composé selon l'une quelconque des revendications 17 et 18, pour servir au traitement d'une hyperglycémie post-prandiale chez des individus diabétiques.

**21.** Composé selon la revendication 1, destiné à servir dans la fabrication d'un médicament pour traiter le diabète chez les mammifères ou pour traiter une hyperglycémie post-prandiale chez des individus diabétiques.

**22.** Composé selon la revendication 2, destiné à servir à fabriquer un médicament pour traiter le diabète des mammifères ou traiter une hyperglycémie post-prandiale chez des individus diabétiques.

**23.** Forme dosée pharmaceutique contenant un composé selon la revendication 1.

**24.** Forme dosée pharmaceutique contenant un composé selon la revendication 2.

**Revendications pour les Etats contractants suivants : ES, GR**

**1.** Procédé pour préparer un composé de formule :

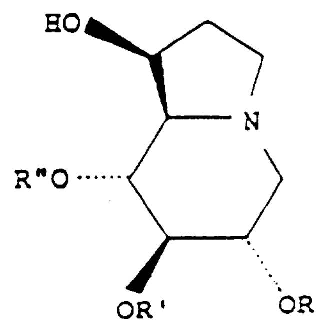

dans laquelle R, R' et R" représentent chacun, indépendamment, un atome d'hydrogène, un groupe alcanoyle en $C_1$-$C_{10}$, cyclohexanecarbonyle,

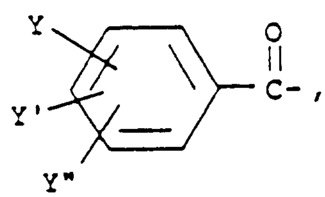

naphtalènecarbonyle éventuellement substitué par un radical méthyle ou halogéno; phényl-(alcanoyle en $C_2$-$C_6$), dans lequel le noyau phényle est éventuellement substitué par un radical méthyle ou halogéno ; un groupe cinnamoyle ; un groupe pyridinecarbonyle éventuellement substitué par un radical méthyle ou halogéno ; un groupe thiophènecarbonyle éventuellement substitué par un radical méthyle ; un groupe furannecarbonyle éventuellement substitué par un radical méthyle ; ou un radical glycosyle, un radical O-méthylglycosyle ou un radical glycosyle acylé Ac et contenant 1 à 3 motifs hexose ou pentose avec fixation en position 1 du radical glycosyle; Y représente un atome d'hydrogène, un groupe alkyle en $C_1$-$C_4$, alcoxy en $C_1$-$C_4$, halogéno, trifluorométhyle, alkylsulfonyle en $C_1$-$C_4$, alkylmercapto en $C_1$-$C_4$, cyano ou diméthylamino ; Y' représente un atome d'hydrogène, un groupe alkyle en $C_1$-$C_4$, alcoxy en $C_1$-$C_4$, halogéno, ou bien il est combiné avec Y pour former un groupe 3,4-méthylènedioxy ; Y" représente un atome d'hydrogène, un groupe alkyle en $C_1$-$C_4$, alcoxy en $C_1$-$C_4$ ou halogéno ; Ac représente un groupe benzoyle ou alcanoyle en $C_2$-$C_6$; le sens des symboles R, R' et R" étant choisi de façon qu'au moins l'un d'entre eux, mais pas plus de deux d'entre eux, représente(nt) un atome d'hydrogène, ou les sels pharmaceutiquement acceptables des composés précités, ce

procédé comprenant la réaction de la castanospermine avec :

(a) l'halogénure ou anhydride d'acide approprié, dans un solvant inerte ; ou bien

(b) l'halogénure ou acétimidate de glycosyle acylé par Ac ou O-benzylé approprié, dans un solvant inerte, opération suivie d'une hydrogénation catalytique pour enlever les groupes benzyle ou éventuellement d'un traitement pour une base pour enlever les groupes Ac ou les autres groupes esters.

**2.** Procédé selon la revendication 1, pour préparer un composé de formule :

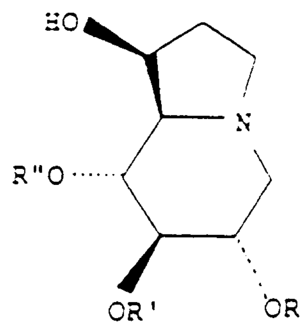

dans laquelle R, R' et R" représentent chacun, indépendamment, un atome d'hydrogène, un groupe alcanoyle en $C_1$-$C_{10}$, cyclohexanecarbonyle,

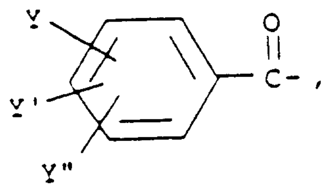

naphtalènecarbonyle éventuellement substitué par titué par un radical méthyle ou halogéno; un groupe phényl-(alcanoyle en $C_2$-$C_6$) dans lequel le noyau phényle est éventuellement substitué par un radical méthyle ou halogéno ; un groupe cinnamoyle, un groupe pyridinecarbonyle éventuellement substitué par un radical méthyle ou halogéno ; un groupe thiophènecarbonyle éventuellement substitué par un radical méthyle ; un groupe furannecarbonyle éventuellement substitué par un radical méthyle ; Y représente un atome d'hydrogène, un groupe alkyle en $C_1$-$C_4$, alcoxy en $C_1$-$C_4$, halogéno, trifluorométhyle, alkylsulfonyle en $C_1$-$C_4$, alkylmercapto en $C_1$-$C_4$, cyano ou diméthylamino ; Y' représente un atome d'hydrogène, un groupe alkyle en $C_1$-$C_4$, alcoxy en $C_1$-$C_4$, ou halogéno ou bien il est combiné avec Y pour former un groupe 3,4-méthylènedioxy ; Y" représente un atome d'hydrogène, un groupe alkyle en $C_1$-$C_4$, alcoxy en $C_1$-$C_4$ ou halogéno; le sens des symboles R, R' et R" étant choisi de façon qu'au moins l'un d'entre eux, mais pas plus de deux d'entre eux, représente(nt) un atome d'hydrogène ; ou les sels pharmaceutiquement acceptables des composés précités, le procédé comprenant la réaction de la castanospermine avec l'halogénure ou anhydride d'acide approprié, dans un solvant inerte.

3. Procédé selon la revendication 1, pour préparer un composé de formule :

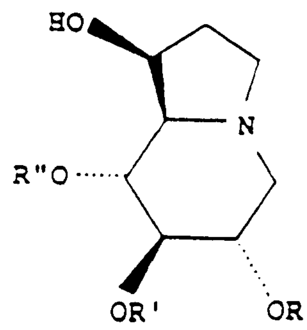

dans laquelle R, R' et R" représentent chacun, indépendamment, un atome d'hydrogène, un groupe alcanoyle en $C_1$-$C_{10}$ ou

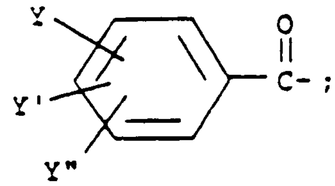

Y représente un atome d'hydrogène, un groupe alkyle en $C_1$-$C_4$, alcoxy en $C_1$-$C_4$, halogéno, trifluorométhyle, alkylsulfonyle en $C_1$-$C_4$, alkylmercapto en $C_1$-$C_4$, cyano ou diméthylamino ; Y' représente un atome d'hydrogène, un groupe alkyle en $C_1$-$C_4$, alcoxy en $C_1$-$C_4$, ou halogéno ou bien il est combiné avec Y pour donner un groupe 3,4-méthylènedioxy ; Y" représente un atome d'hydrogène, un groupe alkyle en $C_1$-$C_4$, alcoxy en $C_1$-$C_4$ ou halogéno ; le sens des symboles R, R' et R" étant choisis de façon qu'au moins l'un d'entre eux, mais pas plus de deux d'entre eux, représente(nt) un atome d'hydrogène ; ou les sels pharmaceutiquement acceptables des composés ci-dessus, le procédé comprenant la réaction de la castanospermine avec l'halogénure ou anhydride d'acide approprié, dans un solvant inerte.

4. Procédé selon la revendication 1, pour préparer un composé de formule :

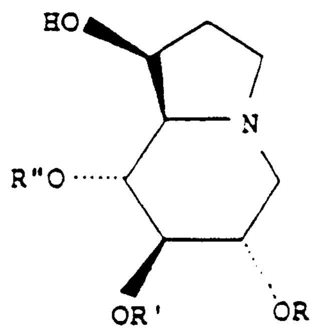

dans laquelle R, R' et R" représentent chacun, indépendamment, un atome d'hydrogène ou

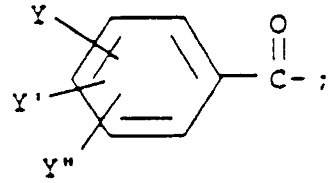

Y représente un atome d'hydrogène, un groupe alkyle en $C_1$-$C_4$, alcoxy en $C_1$-$C_4$, halogéno, trifluorométhyle, alkylsulfonyle en $C_1$-$C_4$, alkylmercapto en $C_1$-$C_4$, cyano ou diméthylamino ; Y' représente un atome d'hydrogène un groupe alkyle en $C_1$-$C_4$, alcoxy en $C_1$-$C_4$, halogéno ou bien il est combiné avec Y pour donner un groupe 3,4-méthylènedioxy ; Y'' représente un atome d'hydrogène, un groupe alkyle en $C_1$-$C_4$, alcoxy en $C_1$-$C_4$ ou halogéno, le sens des symboles R, R' et R'' étant choisi de façon qu'au moins l'un d'entre eux, mais pas plus de deux d'entre eux, représente(nt) un atome d'hydrogène ; ou les sels pharmaceutiquement acceptables des composés précités, le procédé comprenant la réaction de la castanospermine avec l'halogénure ou anhydride d'acide approprié, dans un solvant inerte.

**5.** Procédé selon la revendication 1, pour préparer un composé de formule :

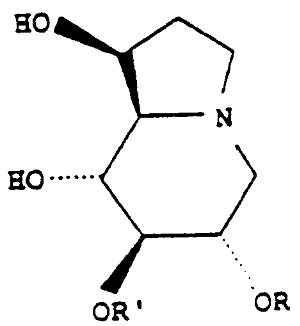

dans laquelle R et R' représentent chacun indépendamment, un atome d'hydrogène ou

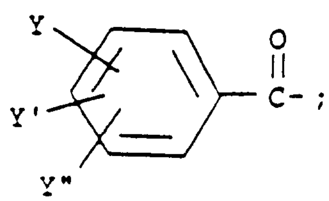

Y représente un atome d'hydrogène, un groupe alkyle en $C_1$-$C_4$, alcoxy en $C_1$-$C_4$, halogéno, trifluorométhyle, alkylsulfonyle en $C_1$-$C_4$, alkylmercapto en $C_1$-$C_4$, cyano ou diméthylamino ; Y' représente un atome d'hydrogène, un groupe alkyle en $C_1$-$C_4$, alcoxy en $C_1$-$C_4$, ou halogéno ou bien il est combiné avec Y pour donner un groupe 3,4-méthylènedioxy ; Y'' représente un atome d'hydrogène, un groupe alkyle en $C_1$-$C_4$, alcoxy en $C_1$-$C_4$ ou halogéno, le sens des symboles R et R' étant choisi de façon qu'au moins l'un d'entre eux représente autre chose qu'un atome d'hydrogène ; ou les sels pharmaceutiquement acceptables des composés précités, le procédé comprenant la réaction de la castanospermine avec l'halogénure ou anhydride d'acide approprié, dans un solvant inerte.

**6.** Procédé selon la revendication 1, pour préparer un composé de formule :

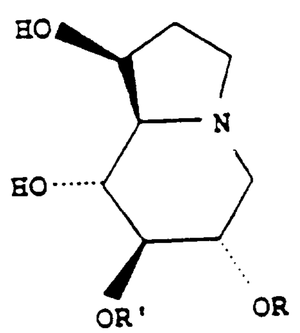

dans laquelle R et R' représentent chacun, indépendamment, un atome d'hydrogène ou

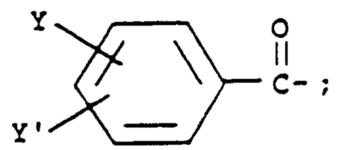

Y représente un atome d'hydrogène, un groupe alkyle en $C_1$-$C_4$, alcoxy en $C_1$-$C_4$, halogéno, trifluorométhyle, alkylsulfonyle en $C_1$-$C_4$, alkylmercapto en $C_1$-$C_4$, cyano ou diméthylamino ; Y' représente un atome d'hydrogène, un groupe alkyle en $C_1$-$C_4$, alcoxy en $C_1$-$C_4$, ou halogéno ou bien Y' est combiné avec Y pour donner un groupe 3,4-méthylènedioxy ; le sens des symboles R et R' étant choisi de façon qu'au moins l'un d'entre eux représente autre chose qu'un atome d'hydrogène ; ou les sels pharmaceutiquement acceptables des composés précités, ce procédé comprenant la réaction de la castanospermine avec l'halogénure ou anhydride d'acide approprié, dans un solvant inerte.

**7.** Procédé selon la revendication 1, pour préparer un composé de formule :

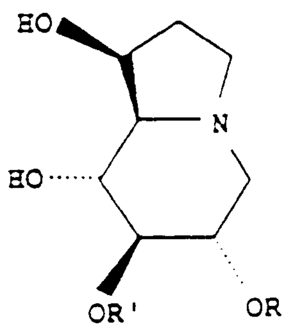

dans laquelle R et R' représentent chacun, indépendamment, un atome d'hydrogène ou

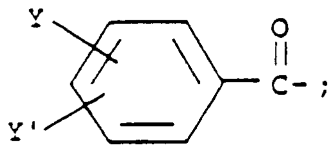

Y représente un atome d'hydrogène ou d'halogène; Y' représente un atome d'hydrogène ou d'halogène ; le sens des symboles R et R' étant choisi de façon qu'au moins l'un d'entre eux représente autre chose qu'un atome d'hydrogène ; ou les sels pharmaceutiquement acceptables des composés précités, le procédé comprenant la réaction de la castanospermine avec l'halogénure ou anhydride d'acide approprié, dans un solvant inerte.

**8.** Procédé selon la revendication 1, pour préparer le 6-benzoate de [1S-(1α,6β, 7α, 8β, 8aβ)]-octahydro-1,6,7,8-indolizinetétrol, qui comprend la réaction de la castanospermine avec le chlorure de benzoyle.

**9.** Procédé selon la revendication 1, pour préparer le 7-benzoate de [1S-(1α,6β, 7α, 8β, 8aβ)]-octahydro-1,6,7,8-indolizinetétrol, qui comprend la réaction de la castanospermine avec le chlorure de benzoyle.

**10.** Procédé selon la revendication 1, pour préparer le 6-(4-fluorobenzoate) de [1S-(1α,6β, 7α, 8β, 8aβ)]-octahydro-1,6,7,8-indolizinetétrol, qui comprend la réaction de la castanospermine avec le chlorure de 4-fluorobenzoyle.

**11.** Procédé selon la revendication 1, pour préparer le 7-(4-fluorobenzoate) de [1S-(1α,6β, 7α, 8β, 8aβ)]-octahydro-1,6,7,8-indolizinetétrol, qui comprend la réaction de la castanospermine avec le chlorure de 4-fluorobenzoyle.

**12.** Procédé selon la revendication 1, pour préparer le 6,8-dibutanoate de [1S-(1α,6β, 7α, 8β, 8aβ)]-octahydro-1,6,7,8-indolizinetétrol, qui comprend la réaction de la castanospermine avec l'anhydride butirique.

**13.** Procédé selon la revendication 1, pour préparer le 6-butanoate de [1S-(1α,6β, 7α, 8β, 8aβ)]-octahydro-1,6,7,8-indolizinetétrol, qui comprend la réaction de la castanospermine avec le chlorure de butyryle.

**14.** Procédé selon la revendication 1, pour préparer un composé de formule :

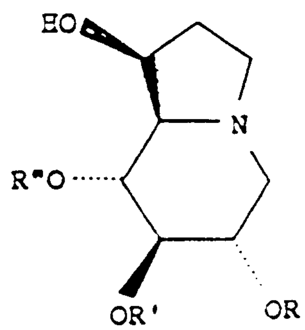

dans laquelle R, R' et R'' représentent chacun, indépendamment, un atome d'hydrogène, ou un radical glycosyle, un radical O-méthylglycosyle ou un radical glycosyle acylé Ac et contenant 1 à 3 motifs hexose ou pentose, avec fixation en position 1 du radical glycosyle ; Ac représente un groupe benzoyle ou un groupe alcanoyle en $C_2$-$C_6$, le sens des symboles R, R' et R'' étant choisi de façon qu'au moins l'un d'entre eux, mais pas plus de deux d'entre eux, représente(nt) un atome d'hydrogène ; ou les sels pharmaceutiquement acceptables des composés précités, le procédé comprenant la réaction de la castanospermine ou d'une castanospermine estérifiée avec l'halogénure ou acétimidate de glycosyle acylée par Ac ou O-benzylé approprié, dans un solvant inerte, ce qui est suivi d'une hydrogénation catalytique pour enlever les groupes benzyles ou éventuellement d'un traitement par une base pour enlever les groupes Ac ou d'autres groupes esters.

**15.** Procédé selon la revendication 1, pour préparer le 8-O-(α-D-glucopyrannosyl)--[1S--(1α,6β,7α,8β,8aβ)]-octahydro-1,6,7,8-indolizinetétrol, qui comprend la réaction du 6,7-dibenzoate de [1S-(1α,6β,7α,8β,8aβ)-]-octahydro-1,6,7,8-indolizinetétrol avec le trichloroacétimidate de 2,3,4,6-tétra-O-(phénylméthyl)-α-D-glucopyrannosyle , opération suivie d'un traitement par une base puis d'une hydrogénation sur du palladium sur du charbon.